(19) Europäisches Patentamt
European Patent Office
Office européen des brevets

(11) EP 2 900 830 B1

(12) EUROPEAN PATENT SPECIFICATION

(45) Date of publication and mention of the grant of the patent:
**09.11.2016 Bulletin 2016/45**

(21) Application number: **13766994.1**

(22) Date of filing: **27.09.2013**

(51) Int Cl.:
***C12Q 1/34*** *(2006.01)*

(86) International application number:
**PCT/EP2013/070248**

(87) International publication number:
**WO 2014/049142 (03.04.2014 Gazette 2014/14)**

# (54) SWITCHABLE REPORTER ENZYMES FOR HOMOGENOUS ANTIBODY DETECTION

UMSCHALTBARE REPORTERENZYME ZUM HOMOGENEN NACHWEIS VON ANTIKÖRPERN

ENZYMES REPORTEURS COMMUTABLES POUR LA DÉTECTION D'ANTICORPS HOMOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.09.2012 US 201261706186 P**

(43) Date of publication of application:
**05.08.2015 Bulletin 2015/32**

(73) Proprietor: **Technische Universiteit Eindhoven
5612 AZ Eindhoven (NL)**

(72) Inventors:
- **BANALA, Sambashiva
Ashburn, Virginia 20147 (US)**
- **APER, Stijn Johannes Amanda
4765 B Zevenbergschen Hoek (NL)**
- **MERKX, Maarten
5685 BR Best (NL)**

(74) Representative: **Kelly, Donal Morgan
FRKelly
27 Clyde Road
Dublin D04 F838 (IE)**

(56) References cited:
**WO-A1-2008/095222 US-A1- 2010 247 529**

- **GOLYNSKIY MISHA V ET AL: "Antibody detection by using a FRET-based protein conformational switch.", CHEMBIOCHEM : A EUROPEAN JOURNAL OF CHEMICAL BIOLOGY 2 NOV 2010, vol. 11, no. 16, 2 November 2010 (2010-11-02), pages 2264-2267, XP002715845, ISSN: 1439-7633**
- **VALLEE-BELISLE A ET AL: "Structure-switching biosensors: inspired by Nature", CURRENT OPINION IN STRUCTURAL BIOLOGY, ELSEVIER LTD, GB, vol. 20, no. 4, 1 August 2010 (2010-08-01) , pages 518-526, XP027234415, ISSN: 0959-440X [retrieved on 2010-06-02]**
- **FAN C ET AL: "Biosensors based on binding-modulated donor-acceptor distances", TRENDS IN BIOTECHNOLOGY, ELSEVIER PUBLICATIONS, CAMBRIDGE, GB, vol. 23, no. 4, 1 April 2005 (2005-04-01), pages 186-192, XP027778076, ISSN: 0167-7799 [retrieved on 2005-04-01]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 900 830 B1

## Description

### FIELD OF THE INVENTION

This invention relates to detection of antibodies for the diagnosis of diseases, immunizations, immune responses, allergies, or the like.

### BACKGROUND OF THE INVENTION

Antibody detection is essential for the diagnosis of many disease states, including infectious diseases, autoimmune diseases and allergies. While a wide variety of analytical techniques have been developed for the detection of antibodies in blood, saliva and other bodily fluids, many of them come with intrinsic limitations such as the requirement for multiple time-consuming incubation steps (ELISA and other heterogeneous, sandwich-type assays), multiple reagents, and/or sophisticated equipment (e.g. surface plasmon resonance).

New generic antibody detection strategies in which molecular recognition and signal generation are integrated within a single protein would be ideal, in particular for high-throughput screening and point-of-care applications. From a protein engineering perspective, the key question is then how antibody binding can be translated into a readily detectable signal change.

One approach for homogenous antibody detection directly in solution is to make use of fluorescently labeled epitopes (or mimotopes) whose fluorescence properties such as intensity, polarization and lifetime are significantly changed upon antibody binding. A sophisticated example is the development of peptide molecular beacons in which peptide epitopes are flanked by two synthetic fluorophores. These probes adopt a rigid and extended conformation upon antibody binding which results in significant changes either in energy transfer between donor and acceptor or pyrene excimer fluorescence. Other FRET-based approaches take advantage of the presence of two identical antigen binding domains, either by inducing the interaction between donor- and acceptor-labeled peptide-oligonucleotides, or vice versa, by a binding-induced disruption of an intramolecular interaction between donor and acceptor fluorescent proteins.

Although these strategies should be generally applicable to detect a wide variety of antibodies, their sensitivity is fundamentally limited by the concentration of fluorescent probe that can be reliably detected. In other words, they lack the enzymatic amplification step that is characteristic of ELISA.

Several groups have explored strategies to couple antibody recognition directly to a change in enzymatic activity. A common approach is to introduce peptide epitopes at permissive sites within reporter enzymes such as β-galactosidase, β-lactamase, alkaline phosphatase and lysozyme. However, these hybrid enzymes are catalytically compromised and analyte binding often results in a further decrease in activity, which is a serious drawback from an application point of view. Even when an increase in activity is observed, allosteric enzymes typically display a relatively modest 2-5 fold change in activity, resulting in high background activities. Finally, since their performance depends on subtle allosteric mechanisms, development of these systems is very much a process of trial-and-error rather than rational design.

Combinatorial approaches such as phage display and *in vivo* selection strategies have been reported in an effort to make development of these allosterically regulated reporter enzymes more efficient, but these approaches have not solved the intrinsic problem of small changes in enzyme activity.

A different strategy that has been pursued with some success is to make use of antibody-induced oligomerization of reporter enzymes or complementation of split reporter enzymes. These approaches utilize the bivalent nature of antibodies to bring together two proteins (or protein-fragments) to form an active enzyme. Unlike the allosterically regulated enzymes, which need to be developed for each new antibody/epitope, these approaches should be applicable to any target antibody, but they also have some intrinsic limitations. For the split enzyme systems, the reconstituted enzyme activity is typically low (only 1-2%) compared to its parent enzyme. Furthermore, the fragments have a tendency to self-associate, which makes the sensor performance dependent on the sensor concentrations and therefore less robust than a single protein sensor.

The present invention provides a different design principle for the development of antibody-responsive reporter enzymes that addresses many of the limitations described *supra*.

### SUMMARY OF THE INVENTION

Detection of antibodies is essential for the diagnosis of many diseases including infectious diseases, autoimmune diseases and allergies. Current heterogeneous assays such as ELISA (enzyme- linked immune sorbent assay) require multiple time-consuming binding and washing steps, which limits their application in low cost point of care diagnostics and high-throughput screening.

With this invention, we present a new approach that allows one-step detection of antibodies directly in solution using a switchable reporter enzyme. The sensor design is highly modular, including the enzyme TEM1-β-lactamase (PDB Accession/Version No 1ZG4_A, GI:67464382) fused to its natural inhibitor protein (BLIP; UniProtKB/Swiss-Prot Accession/Version No P35804.1, GI:543897) via a long, semi-flexible peptide linker. Bivalent binding of antibody to two epitope sequences introduced at the ends of the linker disrupts the enzyme-inhibitor complex, resulting in an increase in enzyme activity that can be monitored using simple colorimetric or fluorescent read outs. Using the anti-HIV1 p17 antibody as an examplary target, the intramolecular affinity for the enzyme-inhibitor was optimized to yield a reporter enzyme whose activity increased 10-fold in the presence of pM concentrations of the target antibody ($Kd$ = 0.17 nM). A reporter enzyme that targets a completely different antibody could be obtained without any further sensor optimization by simply replacing the epitope sequence.

A thermodynamic scheme describing the dependence of sensor performance on the linker properties and the affinities of the antibody-epitope and enzyme-inhibitor pairs was developed and tested by deliberate attenuation of the antibody-epitope interaction. Unlike previous protein engineering approaches based on allosteric modulation of an enzyme active site, our approach provides a generic, modular framework for the rational design of antibody reporter enzymes for homogenous immunoassays.

In one embodiment the invention is an antibody detection method where a biosensor is used for detecting an antibody. The biosensor is an enzyme covalently linked to an inhibitor protein via a peptide linker having two epitopes at the ends of the peptide linker. The biosensor is defined by two equilibrium constants, $K_{closed\text{-}open,1}$ and $K_{closed\text{-}open,2}$ that describe the equilibrium between a closed and an open state of the biosensor in the absence and presence, respectively, of the antibody according to:

$$K_{closed\text{-}open,1} = K_{d(EI)}/C_{eff,(EI)},$$

and

$$K_{closed\text{-}open,2} = 0.5 * K_{d,(EI)}/K_{d(AP)} * C_{eff,(AP)}/C_{eff(EI)}$$

whereby:

- $K_{d(AP)}$ is an intermolecular dissociation constant of a monovalent binding of the antibody and the epitope,
- $K_{d(EI)}$ is an intermolecular dissociation constant of the binding of the enzyme and the inhibitor protein,
- $C_{eff(EI)}$ is an effective concentration of the inhibitor protein in proximity of the enzyme, and
- $C_{eff(AP)}$ is an effective concentration of a free epitope in proximity of the remaining antigen-binding domain of the antibody.

For the biosensor to work in detecting an antibody, $K_{closed\text{-}open,1}$ is smaller than $K_{closed\text{-}open,2}$. In one example, $K_{closed\text{-}open,1}$ is less than 3. In a preferred embodiment, $K_{closed\text{-}open,1}$ is larger than 0 and less than 0.2. Regarding $K_{closed\text{-}open,2}$, in one example $K_{closed\text{-}open,2}$ is greater than 0.2. In another example, $K_{closed\text{-}open,2}$ is larger than 0.2 and less than $10^6$.

The equilibrium constants, $K_{closed\text{-}open,1}$ and $K_{cliosed\text{-}open,2}$, can be determined by measuring the enzymatic activity of the biosensor in the absence (i.e. $K_{closed\text{-}open,1}$) and presence of saturating amount of target antibody (i.e. $K_{closed\text{-}open,2}$) and by comparing the enzymatic activity to that of the same concentration of the enzyme alone.

The $K_{d(AP)}$ can be determined by titration of the antibody to its epitope peptide conjugated to a fluorescent group and monitoring the formation of the antibody-peptide complex by using fluorescence anisotropy. Alternatively, formation of the antibody-peptide complex can be monitored using surface plasmon resonance or isothermal titration calorimetry.

The $K_{d(EI)}$ can be determined by measuring enzymatic activity of the enzyme as a function of substrate concentration (Michaelis Menten kinetics) in the absence and presence of a known concentration of the inhibitor domain. The $K_i$ (= $K_{d(EI)}$) was calculated using $K_M$ (+BLIP) = $K_M$ (1+([BLIP]/ $K_i$)) , which represent the relation between the 2 $K_M$ values, the inhibitor concentration and $K_i$ for a competitive inhibitor. Alternative methods for determining this constant include biophysical methods such as the use of surface plasmon resonance or isothermal titration calorimetry to monitor complex formation between enzyme and inhibitor.

$C_{eff(EI)}$ can be derived after determining $K_{d,\ EI}$ and $K_{closed\text{-}open,1}$ from $K_{closed\text{-}open,1} = K_{d(EI)}/C_{eff,(EI)}$.

$C_{eff(AP)}$ can be derived after determining $K_{d,\ EI}$, $Kd_{,\ AP}$, $C_{eff(EI)}$ and $K_{closed\text{-}open,2}$ $K_{closed\text{-}open,2} = 0.5 * K_{d,(EI)}/K_{d(AP)} * C_{eff,(AP)}/C_{reff(EI)}$.

In another embodiment the invention, an *in vitro* antibody-detecting method is provided. The method entails contacting a sample with a biosensor. The biosensor includes a reporter enzyme (for example, but not limited to, beta-lactamase), an inhibitor domain (for example, but not limited to, beta-lactamase inhibitor protein, BLIP) having affinity for the reporter enzyme, at least two epitopes, whereby each epitope has affinity for the antibody, and a linker. The method further entails determining the activity of the reporter enzyme in the presence of a sample, and attributing the activity of the reporter enzyme in the presence of the sample to the quantitative or qualitative presence or absence of an antibody. Now in the absence of the antibody, the biosensor is in a closed, inactive state in which at least some (e.g. at least 30%, 50% or 80% in different examples) of the reporter enzyme forms an intramolecular complex with the inhibitor domain. In different words, the equilibrium is to the left. A bivalent binding between two antigen binding domains present in the antibody and the (at least) two epitopes present at the ends of the linker between the reporter enzyme and the inhibitor domain in the biosensor changes the equilibrium between the closed (inactive) and open (active) state of the biosensor such that the amount of the reporter enzyme that forms an intramolecular complex with the inhibitor domain is decreased.

Also described is an *in vitro* antibody-detecting method. In this method, a sample is contacted with a biosensor which is displaceable between an open state and a closed state.

The biosensor includes a reporter enzyme (for example, but not limited to, beta-lactamase) or a fragment thereof, an inhibitor domain (for example, but not limited to, beta-lactamase inhibitor protein, BLIP)or a fragment thereof having affinity for the reporter enzyme, at least two epitopes, wherein each epitope has affinity for the antibody, and a linker separating the at least two epitopes. This method further entails determining the activity of the reporter enzyme in the presence of the sample, and attributing the activity of the reporter enzyme in the presence of the sample to the quantitative or qualitative presence or absence of the antibody. Now, in the closed state, the reporter enzyme and the inhibitor domain form an (inactive) intramolecular complex. The binding (e.g. bivalent binding) of an antibody to the at least two epitopes changes the equilibrium between the closed (inactive) and an open (active) state of the biosensor, which thereby displaces the biosensor from the closed state to the open state, such that the amount of reporter enzyme forming an intramolecular complex with the inhibitor domain is decreased.

Also described is a biosensor displaceable between an open state and a closed state. The biosensor includes a reporter enzyme (for example, but not limited to, beta-lactamase) or a fragment thereof, an inhibitor domain (for example, but not limited to, beta-lactamase inhibitor protein, BLIP) or a fragment thereof having affinity for the reporter enzyme, at least two epitopes, whereby each epitope has affinity for the antibody, and a linker separating the at least two epitopes.

In the closed state of this method, the reporter enzyme and the inhibitor domain form an (inactive) intramolecular complex. Further in this method, the binding of an antibody to the at least two epitopes changes the equilibrium between the closed (inactive) and an open (active) state of the biosensor, thereby displacing the biosensor from the closed state to the open state, such that the amount of said reporter enzyme forming an intramolecular complex with the inhibitor domain is decreased.

Optionally, binding of an antibody to the at least two epitopes changes the equilibrium between the closed (inactive) and an open (active) state of the biosensor, which thereby displaces the biosensor from the closed state to the open state, such that the activity of said reporter enzyme is increased.

Optionally, in the presence of an antibody, the biosensor is in the open state. Further optionally, in the presence of an antibody, a bivalent binding between two antigen binding domains present in said antibody and the at least two epitopes displaces the biosensor to the open state. Still further optionally, in the presence of an antibody, a bivalent binding between two antigen binding domains present in the antibody and the at least two epitopes present at the ends of the linker between the reporter enzyme and the inhibitor domain in the biosensor, displaces the biosensor to the open state. Still further optionally, in the open state, the reporter enzyme is spaced apart from the inhibitor domain, thereby allowing activity of the reporter enzyme.

Optionally, in the absence of an antibody, the biosensor is in the closed state. Further optionally, in the absence of an antibody, the biosensor is in the closed state whereby at least some of the reporter enzyme forms an intramolecular complex with the inhibitor domain. Still further optionally, in the closed state, the reporter enzyme and the inhibitor domain form a molecular complex, thereby inhibiting activity of the reporter enzyme.

Optionally, the activity of the reporter enzyme is proportional to the quantitative or qualitative presence or absence of the antibody.

Optionally, the reporter enzyme is a polypeptide capable of catalysing the reaction of a non-visible substrate to a visible product. Optionally, the reporter enzyme is beta-lactamase or a fragment thereof. Further optionally, the reporter enzyme is TEM1 $\beta$-lactamase or a fragment thereof.

Optionally, the inhibitor domain is a polypeptide having affinity for the reporter enzyme and capable of forming a molecular complex with the reporter enzyme to inhibit activity of the reporter enzyme. Optionally, the inhibitor domain is beta-lactamase inhibitory protein (BLIP) or a fragment thereof. Alternatively, the inhibitor domain is an inhibitory peptide derived from phage display.

Optionally, the at least two epitopes are polypeptides or polypeptide fragments having affinity for the antibody and capable of binding, optionally selectively binding, to the antibody, optionally to the antigen-binding fragments of the antibody. Optionally, each epitope is capable of independently binding, optionally independently selectively binding, to the each respective antigen-binding fragment of the antibody.

Optionally, the linker is a polypeptide or polypeptide fragment including at least one flexible block of $(GSG)_6$. Further optionally, the linker further includes at least one α-helical block. Still further optionally, the linker further includes at least one α-helical block having six EAAAK repeats. Still further optionally, the linker further includes two α-helical blocks, each block having six EAAAK repeats.

Optionally, each epitope is located at each respective end of the linker. Further optionally, each of the reporter enzyme and the inhibitor domain is located at each respective end of the linker. Still further optionally, a first epitope and the reporter enzyme are located at a first end of the linker, and a second epitope and the inhibitor domain is located at a second, opposing end of the linker.

## BRIEF DESCRIPTION OF THE DRAWINGS

**FIG. 1** shows according to an exemplary embodiment of the invention a switchable antibody reporter enzyme based on the antibody-induced disruption of an intramolecular complex between enzyme and inhibitor domains. (**E** = enzyme; **I** = inhibitor; **S** = substrate; **P** = product).

**FIGs. 2A-C** show according to an exemplary embodiment of the invention: (**2A**) shows a general structure of the antibody reporter enzymes. **Abs-1-3 (SEQ ID NO: 12,13, and 21 respectively)** contain epitopes targeting the anti-HIV1-p17 antibody, **Abs-4 (SEQ ID NO: 40)** contains an HA-tag for binding anti-HA-tag antibodies.

(**2B**) shows a comparison of the β-lactamase activity of β-lactamase alone, **Abs-1 (SEQ ID NO: 12),** and **Abs-2 (SEQ ID NO: 13)** in the absence and presence of 200 nM anti-HIV1-p17 antibody.

(**2C**) shows a quantification of the data shown in **FIG. 2B.** Activity assays were done using 0.3 nM enzyme with 50 μM nitrocefin in 50 mM phosphate buffer (pH 7.0) containing 100 mM NaCl and 1 mg/mL BSA.

**FIG. 3** shows according to an exemplary embodiment of the invention enzymatic activities of **Abs-3** variants **(SEQ ID NOs: 26-35)** (0.1 nM) combining the E104D mutation in the -lactamase with various mutations in BLIP in the absence (**white bars**) and presence (**dashed bars**) of saturating amount (100 nM) of anti-HIV1-p17 antibody. All assays were done using 50 μM nitrocefin in 50 mM phosphate buffer (pH 7.0) containing 100 mM NaCl and 1 mg/mL BSA.

**FIGs. 4A-C** show according to an exemplary embodiment of the invention enzymatic activities of **Abs3-1 (SEQ ID NO: 22) (4A)** and **Abs3-2 (SEQ ID NO: 37) (4B)** as a function of anti-HIV1-p17 antibody concentration. **FIG. 4C** shows **Abs3-2 (SEQ ID NO: 37)** response with anti-HIV1- p17 (10 nM) alone, together with 0.1 mg/mL IgG mix (660 nM) and only with IgG mix (660 nM). **Abs3-2** single epitope variants response towards the anti-HIV1-p17. All assays were done using 50 μM nitrocefin in 50 mM phosphate buffer (pH 7.0) containing 100 mM NaCl and 1 mg/mL BSA. In all the assays sensor concentration was 0.1 nM. (Ab = antibody).

**FIG. 5** shows according to an exemplary embodiment of the invention enzymatic activity of **Abs-4 (SEQ ID NO: 40)** (0.1 nM) with different anti-HA antibody concentration. From the data a $K_d$ value (0.20 nM) was obtained. Response towards the non-specific antibodies (IgG mix) is also shown. All assays were done using 50 μM nitrocefin in 50 mM phosphate buffer (pH 7.0) containing 100 mM NaCl and 1 mg/mL BSA.

**FIG. 6** shows according to an exemplary embodiment of the invention **Abs3-1 (SEQ ID NO: 22)** response with saturating amount of anti-HIV1-p17 in buffer and serum with both nitrocefin and fluorescent substrates (CCF2-FA).

(**6A**) shows an activity assay with nitrocefin (50 μM) in buffer.

(**6B**) shows an assay with 1 μM CCF2- FA in buffer. In both cases 0.1 nM of the **Abs3-1 (SEQ ID NO: 22)** was used.

(**6C**) shows **Abs3-1 (SEQ ID NO: 22)** (5 nM) response in 10% FBS with 10 μM CCF2-FA in the presence and absence of the antibody.

(**6D**) shows a quantification of the activities in **6A, 6B** and **6C.** The maximum activity in the presence of antibody was taken as 100%.

**FIG. 7** shows a thermodynamic model according to an exemplary embodiment of the invention. The thermodynamic model describes the bivalent binding between reporter enzyme and antibody in 3 steps. In **step 1,** antibody binds to one of the two epitopes in a intermolecular fashion ($K_{d,\ AP}$ = intermolecular dissociation constant of the monovalent binding of antibody and epitope). In **step 2,** dissociation of the enzyme-inhibitor complex occurs. $K_{d,\ EI}$ = intermolecular dissociation constant of the binding of enzyme and inhibitor, $C_{eff, EI}$ = effective concentration of inhibitor in proximity of the enzyme. In **step 3,** antibody binds to second epitope of the sensor. $C_{eff, AP}$ = effective concentration of free epitope in proximity of the remaining antigen binding domain of the antibody. Equations (1)-(4) are shown in **FIG. 7.**

**FIG. 8** shows according to an exemplary embodiment of the invention activity change of **Abs2-2 (SEQ ID NO: 20)** (0.3 nM) as a function of anti-HIV1-p17 concentration. From the data a *Kd* value (108 nM) was obtained. **Abs3-2 (SEQ ID NO: 37)** (0.1 nM) activity change with the antibody concentration was also shown for comparison.

**FIG. 9** shows according to an exemplary embodiment of the invention enzymatic activities of **Abs-5 (SEQ ID NO: 42)** as a function of antibody concentration. The solid lines represent a fit to **Eq. 2,** yielding a $K_d$ value of 1.13 nM $\pm$ 0.46.

**FIG. 10** shows according to an exemplary embodiment of the invention the affinity of the short HIV1-p17 epitope (WEKIRLR; **(SEQ ID NO: 1).**

**FIGs. 11-12** show fluorescence polarization assays according to exemplary embodiments of the invention. **FIG. 11** shows titration of 10 nM ELDRWEKIRLRP-GGG-C(fluorescein) with HIV1-p17 antibody monitored using fluorescence polarization. The data were fit to **Eq. 8** yielding an apparent $K_d$ = 24$\pm$3 nM. **FIG. 12** shows a competition assay with unlabelled peptide. Complex of antibody and fluorescein-labeled peptide (100 nM each) was titrated with non-labeled peptide (ELDRWEKIRLRP; **(SEQ ID NO: 3).** Fitting the data to **Eqs. 9** and **10** yielded a $K_d$ value of 42$\pm$1 nM.

**FIGs. 13-14** show fluorescence polarization assays according to exemplary embodiments of the invention. **FIG. 13** shows titration of 2 nM YPYDVPDYA-GGG-C(fluorescein) with anti-HA antibody monitored using fluorescence polarization. The data were fit to **Eq. 8** yielding a $K_d$ = 0.58$\pm$0.22 nM. **FIG. 14** shows a competition assay with unlabeled peptide. Complex of antibody and fluorescein-labeled peptide (10 nM each) was titrated with non-labeled peptide (YPYDVPDYA; **(SEQ ID NO: 5).** Fitting the data to **Eqs. 9** and **10** yielded a $K_d$ value of 4.5$\pm$1 nM.

**FIG. 15** shows according to exemplary embodiments of the invention titration of 10 nM (EHKYSWKS-GGG-C(fluorescein)) with anti-Dengue-1 antibody monitored using fluorescence polarization. The data were fit to **Eq. 8** yielding a $K_d$ of 70 $\pm$ 13 nM.

**FIG. 16** shows according to exemplary embodiments of the invention enzymatic activity of **Abs-3** mutants (0.3 nM) in the presence and absence of 200 nM of the anti-HIV1-p17 antibody. Antibody and sensor were incubated for 1 h at RT. Then 50 $\mu$M of nitrocefin was added and the measurement was started immediately. The assay was performed in pH 7.0 phosphate buffer (50 mM) that contains NaCl (100 mM) and BSA (1 mg/mL).

**FIGs. 17-18** show Michaelis-Menten plots showing the activity of beta-lactamase-E104D (0.1 nM) as function of nitrocefin concentration in the absence and presence of 5 $\mu$M BLIP-E31A **(SEQ ID NO: 47; FIG. 17),** or 5 $\mu$M BLIP-F142A **(SEQ ID NO: 48; FIG. 18).** The solid lines represent fits to the Michaelis-Menten equation: **FIG. 16:** $K_M$= 62 $\mu$M and $K_M$ (+BLIP-E31A) = 210 $\mu$M; *K*i = 2.1 $\mu$M. **FIG. 17:** $K_M$ = 88 $\mu$M and $K_M$ (+BLIP-F142A) = 239 $\mu$M; *K*i = 2.9 $\mu$M.

**FIG. 19** show the primers for the mutagenesis according to an exemplary embodiment of the invention.

**FIG. 20** show the primers used for generation of **Abs-4 (SEQ ID NO: 40) and Abs-5 (SEQ ID NO: 42)** according to an exemplary embodiment of the invention.

**FIG. 21** show enzymatic activity of Abs-2 mutants (0.3 nM) in the presence and absence of 200 nM of the target antibody according to an exemplary embodiment of the invention.

## DETAILED DESCRIPTION

The switchable reporter enzymes can have a full length reporter enzyme that is conjugated to an inhibitor domain via a long semi-flexible linker, forming a catalytically inactive enzyme-inhibitor complex in the absence of its target antibody (**FIG. 1**). Peptide epitopes specific to the antibody of interest are introduced in the linker, one is next to the enzyme and the other adjacent to the inhibitor. Binding of a single antibody to both epitopes separates the enzyme-inhibitor complex, resulting in an increase in enzyme activity.

The feasibility of the technology of this invention was demonstrated using β-lactamase as a reporter enzyme. The affinity between this β-lactamase and its inhibitor protein BLIP was designed to yield single-protein reporter enzymes that allow detection of pM concentrations of specific antibodies using simple colorimetric or fluorescent read-outs. Moreover, because of the modular architecture of theses sensors, we show that epitope sequences can be readily exchanged without compromising the sensors' performance.

**Sensor Design**

**FIG. 2A** shows the general design of our antibody-reporter enzymes. In this example, TEM1 β-lactamase was chosen as a reporter enzyme because it does not require oligomerization for activity and many substrates are available both for colorimetric and fluorescence detection. The enzyme has been a popular target for testing new protein engineering concepts, including sensors for antibody detection based on subtle allosteric regulation of enzyme activity. Most importantly, a variety of inhibitors are available for TEM1 β-lactamase, ranging from relatively weak inhibitory peptides derived from phage display ($K_i$ ~ 140 μM) to the natural β-lactamase inhibitor protein (BLIP; **(SEQ ID NO:46**), which has a $K_i$ of 0.5 nM.

In an exemplary design we focused on developing a sensor for the detection of the anti-HIV1-p17 antibody. Several well-characterized linear epitope sequences are available for this antibody, which has made it a popular choice for the development of new homogeneous antibody detection assays. The linker between the enzyme and the inhibitor modules initially has two short peptide epitopes (WEKIRLR; **(SEQ ID NO:** 1), *Kd*~ 3μM; **FIG. 10)** specific for the HIV1-p17-antibody that are separated by three flexible blocks of $(GSG)_6$ and two α-helical blocks each having six EAAAK **(SEQ ID NO: 7)** repeats. This linker was also used in a recently reported FRET sensor protein based on the same switching strategy (Golynskiy, M. V., Rurup, W. F., and Merkx, M. (2010) Antibody detection by using a FRET-based protein conformational switch, ChemBioChem 11, 2264-2267). This study showed that introduction of two 45 Å-helical blocks in the flexible linker was essential for the linker to efficiently bridge the distance between the two antigen binding sites.

To establish the influence of inhibitor affinity, two variants were constructed in an exemplary embodiment containing either the weak-binding RRGHYY **(SEQ ID NO: 8)** peptide **(Abs-1; SEQ ID NO: 12)** or the strong BLIP protein as inhibitor domain **(Abs- 2; (SEQ ID NO: 13).** To allow proper folding, proteins were expressed in *E. coli* BL21 (DE3) using a periplasmic leader sequence and purified using an N-terminal His-tag and a C-terminal Strep-tag. This two-step purification protocol ensures the isolation of full length protein only, without truncated version of the sensor lacking e,g. the inhibitor domain.

Enzymatic activity assays using the colorimetric substrate nitrocefin showed that the activity of **Abs-1 (SEQ ID NO: 12)** was similar to that of TEM1 β-lactamase in the absence of any inhibitor **(FIGs. 2B-C).** Moreover, no increase in enzymatic activity was observed upon addition of 200 nM anti-HIV1-p17. These results show that the affinity of the peptide inhibitor used in **Abs-1 (SEQ ID NO: 12)** is too weak to result in substantial enzyme inhibition in the absence of antibody. In contrast, the enzymatic activity of **Abs-2 (SEQ ID NO: 13)** was strongly inhibited compared to that of TEM1 β-lactamase, but no increase in activity was observed upon addition of 200 nM of the target antibody **(FIGs. 2B-C).** Introduction of several single and double point mutations in BLIP also did not show any response to the target antibody **(FIG. 21).** This result suggests that the intramolecular interaction between wild-type BLIP (and its mutants) and β-lactamase domains is actually too strong and that formation of a second epitope-antigen binding domain interaction is not sufficient to overcome this interaction.

**Design of intramolecular enzyme-inhibitor interaction**

To provide a stronger driving force for disrupting the enzyme-inhibitor interaction, **Abs-3** was created in which the short WEKIRLR **(SEQ ID NO: 1)** epitope was extended to a longer epitope (ELDRWEKIRLRP; **SEQ ID NO: 3;** $K_d$ = 42 nM; **FIGs. 11-12).** In addition, to systematically attenuate the interaction between the β-lactamase and BLIP, a series of **Abs-3** variants was explored with mutations in either BLIP, β-lactamase, or both. The X-ray structure of the TEM1 β-lactamase-BLIP complex has been reported, and for many of the residues at the binding interface their contribution to the binding strength has been determined (see **(A)** Zhang, Z., and Palzkill, T. (2003) Determinants of binding affinity and specificity for the interaction of TEM-1 and SME-1 beta-lactamase with beta-lactamase inhibitory protein, The Journal of biological chemistry 278, 45706-45712, and **(B)** Natalie C.J. Strynadka, Susan E. Jensen, Pedro M. Alzari & Michael N.G. James (1996) A potent new mode of beta-lactamase inhibition revealed by the TEM-1/BLIP complex at 1.7 A resolution. Nature Structural & Molecular Biology 3, 290 - 297).

First, several single point mutations were introduced in BLIP. These mutations were previously reported to have affinities ranging from 20-150 nM, but none of them yielded antibody-responsive **Abs-3** variants **(FIG. 16).** A similar result was obtained for a single point mutation in β-lactamase (E104D; **SEQ ID NO: 45),** which in a recent study was reported to have a 3 orders of magnitude lower affinity for BLIP than wt β-lactamase ($K_i$ = 1500 nM) (Hanes, M. S., Reynolds, K. A., McNamara, C., Ghosh, P., Bonomo, R. A., Kirsch, J. F., and Handel, T. M. (2011) Specificity and cooperativity at beta-lactamase position 104 in TEM-1/BLIP and SHV-1/BLIP interactions, Proteins 79, 1267-1276).

However, combination of the E104D mutation in β- lactamase and single point mutations in BLIP yielded several sensor variants that showed an increase in enzymatic activity upon addition of the target antibody **(FIG. 3).** The variants that displayed substantial activity in the absence of antibody typically showed only a modest increase in activity upon antibody binding. The two most promising variants, Abs-3-E104D/E31A **(Abs3-1; SEQ ID NO: 22)** and Abs-3-E104D/F142A **(Abs3-2; SEQ ID NO: 37),** were further characterized, as these showed low background activity and a 5-6 fold increase in enzyme activity. To determine the affinity of each sensor for their target antibody, the rate of nitrocefin hydrolysis was measured as a function of antibody concentration using 100 pM of sensor **(FIGs. 4A-B).**

Fitting these curves assuming a 1:1 binding model yielded dissociation constants of 0.17±0.03 nM for **Abs3-1 (SEQ ID NO: 22)** and 0.19±0.02 nM for **Abs3-2 (SEQ ID NO:37)**, which is 200-fold lower than that of a single epitope peptide ($K_d$ = 42 nM). The bivalent interaction between sensor and antibody thus not only provides a convenient switching mechanism, but also results in a substantial increase in overall affinity. In further testing of this mechanism we verified that both epitopes are required for antibody-induced activation. Two variants of **Abs3-2** were generated in which either the epitope next to the enzyme (Abs-3E0E2; **SEQ ID NO: 38)** or the epitope adjacent to the BLIP domain were deleted (Abs-3E2E0; **(SEQ ID NO: 39).** Indeed, both variants showed a low enzymatic activity and none of them showed any increase in enzymatic activity up to 100 nM of anti- HIV1-p17 antibody (**FIG. 4C**). These results confirmed that the activity increase is due to bivalent binding of the antibody to the two epitope sequences. To test the specificity of the sensors, **Abs3-2** (and **Abs3-1**) was also incubated with a random mix of IgG proteins. No significant increase in enzyme activity was observed up to the highest concentration of IgG tested (2 μM). Moreover, the presence of non-specific IgG's did also not affect the binding of the target antibody, as a similar increase in enzyme activity was observed upon addition of 10 nM anti-HIV1-p17 in the absence and presence of a large excess of IgG mix (0.1 mg/mL, i.e.660 nM) **(FIG. 4C).** The latter is important because in serum specific antibodies need to be detected against a background concentration of non-binding antibodies.

## Targeting a different antibody by exchanging epitopes

To challenge the modularity of our sensor design we tested whether the epitope sequences could be exchanged for epitope sequences targeting a different antibody. A reporter enzyme targeting an HA-tag-specific antibody **(Abs-4; SEQ ID NO: 40)** was constructed by replacing the epitope sequences present in **Abs3-1** by YPYDVPDYA **(SEQ** ID **NO: 9).** The monovalent affinity of the anti-HA-antibody for this peptide was found to be ~5 nM based on fluorescence polarization titration experiments (**FIGs. 13-14),** which is similar to that of the anti-HIV1p17 antibody for the long epitope sequences present in **Abs-3 (SEQ ID NO: 21).** Although no sensor optimization was performed, **Abs-4 (SEQ ID NO: 40)** showed very similar sensor properties compared to its parent sensor **Abs3-1 (SEQ ID NO:22).** Titration of anti-HA antibody again resulted in 7-fold increase in activity and $K_d$ of 0.20 nM for the sensor-antibody interaction **(FIG. 5).** These results show that the framework developed for the exemplary anti-HIV1-p17 antibody can be used to develop β-lactamase reporter enzymes for other antibodies *without* the cumbersome optimization/screening procedures required by previous protein engineering strategies.

## Assays with fluorescent substrate

The use of nitrocefin and other colorimetric substrates for our antibody sensors provide a straightforward means to detect sub-nM concentrations of a specific antibody directly by eye. However, assays based on light absorption measurements require relatively high concentrations of substrate. Since the substrate and BLIP compete for the same binding site on β-lactamase, using high substrate concentrations will result in a relatively high background activity. We therefore assessed the performance of **Abs3-1 (SEQ ID NO: 22)** using the commercially available fluorescent substrate CCF2-FA, which could be used at a 50-fold lower concentration. When this FRET probe is hydrolyzed by the enzyme, a fluorescein molecule (acceptor) is expelled from the probe which results in increase in coumarin fluorescence (donor). Unlike nitrocefin, which even in the absence of sensor is slowly hydrolyzed, CCF2-FA was found to be completely stable providing a low background **(FIG. 6B).** Moreover, the enzymatic activity of the sensor protein in the absence of target antibody was found to be significantly lower using 1 μM of CCF2-FA compared to assays with 50 μM nitrocefin **(FIGs. 6A-B** and **6D).** Therefore using CCF2- FA as a substrate resulted in an increased dynamic range (9-fold) by suppressing the background reaction. The fluorescent substrate also proved essential to employ the reporter enzyme in serum. Unlike nitrocefin, which was rapidly hydrolyzed even in the absence of any reporter enzyme, CCF2-FA was found to be completely stable in both bovine and human serum. Assays using β-lactamase-E104D **(SEQ ID NO: 45)** showed that the enzyme is substantially less active in serum compared to PBS, suggesting the presence of inhibitory compounds in serum. To compensate for this decreased activity, assays in serum were done using 5 nM of reporter enzyme. **FIG. 6C** shows that the dynamic range of the reporter enzyme in serum is at least as high as observed in buffer, showing a 10-fold increase in enzyme activity upon addition of 50 nM of target antibody.

## Thermodynamic model

To get a better insight into the factors that determine the performance of these sensors, a thermodynamic model was derived that describes the bivalent binding between the antibody and the sensor in 3 steps **(FIG. 7).** The first reaction is binding of one of the antigen binding domains to one of the epitope sequences. This equilibrium is determined by the affinity between the epitope and the antigen binding domain ($K_{d,\ AP}$). The second step is dissociation of the β-lactamase-inhibitor complex. This equilibrium depends on the affinity of the enzyme-inhibitor complex ($K_{d,\ EI}$) and the effective concentration of BLIP relative to its enzyme domain ($C_{eff,\ EI}$), which in turn will depend on the linker length and stiffness and the distance that the linker bridges in the complex form. This equilibrium determines how much of the enzyme is inhibited in absence of antibody. If $K_{d,\ EI} > C_{eff,EI}$ than most of the sensor will be already active in the absence of antibody, and a poor sensor is obtained. This was the case for Abs1 and some of the mutants depicted in **FIG. 3.** (e.g. the variant with W150A in the BLIP domain). To have a useful sensor $K_{d,\ EI}/\ C_{eff,\ EI}$ should be preferably be below 0.2, which corresponds to 83% inhibition in the absence of the antibody. The final reaction step is formation of the second epitope-antibody interaction, which again is a function of an effective concentration term ($C_{eff,\ AP}$) and the affinity of the epitope-antibody interaction ($K_{d,\ AP}$). The overall dissociation constant of the reporter enzyme for its target antibody is the product of the equilibrium constants for the three steps and is described by **Eq. 4** in **FIG. 7.**

Of the four parameters that determine $K_{d\text{-overall}}$, two can be determined independently. Fluorescence polarization was used to determine the affinity between a fluorescently labeled epitope peptides and the anti-HIV1 antibody, yielding a $K_{d,\ AP}$ of 42 nM. The dissociation constant for the enzyme-inhibitor pairs was obtained by from enzyme kinetics experiments by determining the competitive inhibition constant for BLIP-E31A and BLIP-F142A and β-lactamase E104D, yielding $K_i$ values of 2.11 μM for BLIP-E31A is and 2.94 μM for BLIP-F142A **(FIG. 18).** Using $K_{d,\ AP}$ = 42 nM and $K_{d,\ EI}$ = 2.9 μM, and $K_{d,\ overall}$ = 0.17 nM, allows one to calculate $C_{eff,\ EI}/C_{eff,\ AP}$) to be 0.56. Since this value is close to 1, this means that the linker itself does not preferentially stabilize either the open or the closed complex, but that the equilibrium between open and closed forms depends on the relative affinities of the enzyme-inhibitor interaction and the epitope-antibody interaction.

**Eq. 4 (FIG. 7)** predicts that the overall affinity of the sensor strongly depends on strength of the antibody-epitope interaction. To test this, we changed the large epitope used in **Abs3-2 (SEQ ID NO: 37)** for the shorter WEKIRLR epitope, which has a $K_d$ of 3.3 μM. The enzyme activity of this variant **(Abs2-2; SEQ ID NO: 20)** also increased upon addition of antibody, but the increase was only 1.8 fold **(FIG. 8).** Moreover, the dissociation constant of the reporter enzyme was increased to 108 nM. This 635-fold increase is roughly consistent with **Eq. 4 (FIG. 7),** which would predict in increase of $(3.3E^{-6}/4.2E^{-8})^2$ = 1900-fold for this substitution. The model also explains the very modest increase in enzyme activity. The activity of **Abs2-2 (SEQ ID NO: 20)** in the absence of its target antibody is described by **Eq. 5-1** (equal **to Eq. 2 in FIG. 7**).

$$K_{closed\text{-}open,1} = K_{d(EI)}/C_{eff,(EI)} \qquad (5\text{-}1)$$

Since this equilibrium depends only on $K_{d(EI)}$ and $C_{eff(EI)}$, the background activity of **Abs2-2 (SEQ ID NO: 20)** and **Abs3-2 (SEQ ID NO: 37)** are very similar. What is different is the equilibrium between the closes and open states of the reporter enzyme in the presence of the saturating amounts of the target antibody. This equilibrium is determined by **steps 2** and **3 (FIG. 7). Eq. 5-2** describes the equilibrium constant for the product of these two steps **2** and **3.**

$$K_{closed\text{-}open,2} = 0.5 * K_{d,(EI)}/K_{d(AP)} * C_{eff,(AP)}/C_{eff(EI)} \qquad (5\text{-}2)$$

Using $C_{eff(EI)}\ /\ C_{eff(AP)}$ = 0.56, $K_{d(EI)}$ = 2.9 μM, and $K_{d(AP)}$ = 3.3 μM yields $K_{closed\text{-}open}$ = 0.78. So even in the presence of saturating concentrations of antibody a significant amount (60%) of the **Abs 2.2 (SEQ ID NO: 20)** reporter enzymes is still in the closed state, which explains the modest increase in enzyme activity observed for this reporter enzyme. The reasonable agreement between the predictions of our thermodynamic model and the experimental results suggest that in first approximation the sensor properties of the β-lactamase-BLIP system can be predicted based on the relative stabilities of the epitope-antibody and the β-lactamase-inhibitor complexes. This model provides useful guidelines for the construction of new reporter enzymes, either using the present β-lactamase-BLIP system or systems based on alternative enzyme-inhibitor pairs. E.g. ideally, $K_{closed\text{-}open,1}$ (in the absence of the antibody) should be below 0.2, whereas $K_{closed\text{-}open,\ 2}$ (in the presence of antibody) should be above 5.

To further challenge the modularity of the biosensor design we tested whether the original epitope sequences could be exchanged for epitope sequences targeting a Dengue type I specific antibody. This reporter enzyme **Abs-5 (SEQ ID NO: 42)** was constructed by replacing the epitope sequences present in **Abs-3-1 (SEQ ID NO: 36)** by EHKYSWKS **(Abs-5).** Fluorescence polarization titration experiments yielded a $K_d$ values of 70 nM for the monovalent peptide-antibody interaction ($K_{d,\ AP}$) **(FIG. 15).** Although no sensor optimization was performed, **Abs-5 (SEQ ID NO: 42)** showed very similar sensor properties compared to its parent sensor **Abs-3-1 (SEQ ID NO: 36).** A 7-fold increase in enzymatic activity was observed upon titration of Dengue type I antibody to **Abs-5 (SEQ ID NO:42),** consistent with a Kd of 1.13 nM ± 0.46 **(FIG. 9).** Although the antibody affinity is slightly attenuated in **Abs-5,** this $K_d$ still reflects a 60-fold increase in affinity compared to the monovalent interaction between antibody and peptide epitope. These results show that the framework developed for the HIV1-p17 antibody allows the antibody specificity to be changed merely by replacing the epitope sequences without the subsequent sensor optimization required by other by protein engineering strategies.

## Experimental Section

### Cloning and mutagenesis

Synthetic DNA sequences encoding the **Abs-1 (SEQ ID NO:12),** BLIP and linker 2 (linker with longer epitope sequence, E2) were ordered from Genscript (Piscataway, USA). Inhibitor peptide in the **Abs-1** was replaced with BLIP sequence by cloning with NcoI and EcoRI restriction enzymes to generate **Abs-2 (SEQ ID NO: 13).** Linker 1 (linker with short epitope sequence, E1) in the **Abs-2** construct was replaced with the linker 2 sequence by cloning with SpeI and NcoI restriction enzymes to generate **Abs-3 (SEQ ID NO: 21)** sensors. **Abs-4 (SEQ ID NO: 40)** and **Abs-5 (SEQ ID NO: 42)** were constructed from **Abs-3** using a strategy described previously (Quan, J. and Tian, J. (2009) Circular polymerase extension cloning of complex gene libraries and pathways. PLoS One 4, e6441). Briefly, the **Abs-3 (SEQ ID NO: 21)** vector was opened with PCR using primers HA-open-FW and HA-open-RW for **Abs-4** and Deng1-open-FW and Deng1-open-RW for **Abs-5.** The linker part of **Abs-3** without HIV1 epitopes was PCR-amplified with the HA-linker-FW and HA-linker-RW primers for **Abs-4** and Deng1-linker-FW and Deng1-linker-RW for **Abs-5.** This PCR-generated linker contains sequences encoding for either the HA-epitope or Dengue-1 epitopes and sequences that overlap with the opened vector. After agarose gel purification, both opened vector and linker were mixed and PCR was performed. The PCR mixture was treated with DpnI to remove any remaining parental DNA. Transformation and then sequencing of colonies showed successful exchange of the epitope sequences. All constructs were cloned into pET29a vectors. The QuikChange site-directed mutagenesis kit (Stratagene) was used in accordance with the manufacturer's instructions to introduce the mutations of interest. All cloning and mutagenesis results were confirmed by DNA sequencing (BaseClear, Leiden, The Netherlands).

### Protein expression and purification

All proteins were expressed and purified using standard protocols. Briefly, *E. coli* BL21(DE3) cells were transformed with the appropriate pET29a vector. The bacteria containing plasmid DNA was grown in LB (2 L) media at 37 degrees Celsius and induced at $OD_{600}$ ~ 0.6 with isopropyl- β-D-thiogalactoside (IPTG; 0.3mM). Induced cells were grown overnight at 15 degrees Celsius, and harvested for 10 min at 8000g. The protein is located in periplasm that was extracted by osmotic shock method. The bacterial pellet was resuspended in 100 mL 30 mM Tris/HCl (pH 8.0), 20% (w/v) sucrose, and 1 mM EDTA, and incubated at room temperature for 10 min under continuous shaking. After centrifugation for 10 min at 8000g, the pellet was resuspended in 100 mL of ice-cold 5 mM $MgSO_4$. After incubation for 10 min at 4 degrees Celsius with continuous shaking, the suspension was centrifuged for 20 min at 12000g. The supernatant (contains the periplasmic protein fraction) was adjusted to pH 7.4 by adding 2 mL of 1 M Tris/HCl (pH 7.4). The supernatant was first loaded onto an immobilized metal-affinity column packed with His-bind resin in accordance with the manufacturer's instructions (Novagen). The eluted fractions were further purified on a Strep-Tactin superflow column (IBA) according to the instructions of the supplier. The purified proteins were dialyzed against 50 mM Tris/HCl (pH 7.1) containing 150 mM NaCl using 3.5 kD MWCO membranes (Spectra/Por 3). The proteins were quantified using a Nanodrop ND-1000 spectrophotometer (Wilmington, USA) by using extinction coefficient at 280 nm (calculated from protein sequence using http://web.expasy.org/protparam/). Protein aliquots were stored at -80 degrees Celsius.

### Activity assays

Antibodies were purchased from commercial sources, anti-HIV-1-p17 (clone 32/1.24.89) from Zeptometrix, HA monoclonal antibody (clone 2-2.2.14) from Thermo Scientific and anti-Dengue virus type I antibody (clone 15F3-1) from Merck Millipore. Nonspecific IgG mix isolated from human serum was purchased from Sigma-Aldrich. Fetal bovine serum (FBS) was purchased from BioChrom AG, Germany. For activity assays, antibody was incubated with sensor proteins for 15 min at room temperature, and then 50 μM of nitrocefin was added. For responsive sensors, 100 pM of sensor was treated up to 100 nM of antibody. For non-responsive sensors 300 pM of sensor was treated with up to 200 nM antibody and incubated for 1 h. The assays were performed in 50 mM phosphate buffer (pH 7.0, containing 100 mM NaCl and 1 mg/mL BSA). Measurements were performed in 96-well plates. Both absorbance (486 nm) with nitrocefin substrate, and fluorescence with CCF2-FA (Ex. 409 nm and Em. 447 nm) was recorded on Safire2 spectrofluoremeter (Tecan). The data were plotted and analyzed using Graphad Prism5 software. Assays using β-lactamase-E104D showed that the enzyme is substantially less active in serum compared to PBS, suggesting the presence of inhibitory compounds in serum. To compensate for this decreased activity, assays in serum were done using 5 nM of reporter enzyme.

**Antibody titrations and $K_d$ measurements**

Antibody titrations were performed as mentioned above, initial 10 min data was used for calculating hydrolysis rate of the substrate. The data, activity change with the antibody concentration, was fit to **Eq. 6** to obtain dissociation constants. In **Eq. 6,** A and B are constants, and [sensor] and [Ab] are the total sensor and antibody concentrations, respectively.

$$\text{Hydrolysis rate} = A \times (([\text{sensor}] + [\text{Ab}] + K_d) - (([\text{sensor}] + [\text{Ab}] + K_d)^2 - 4[\text{Ab}][\text{sensor}])^{1/2}) + B \qquad (6)$$

**Enzyme inhibition assays and *Ki* determination:** To a 100 pM of β- lactamase-E104D mutant 5 μM BLIP protein was added and incubated for 2 h at 30 degrees Celsius. Aliquots of enzyme alone or enzyme-inhibitor complex was pipetted into a 96-well plate. To these samples different concentration of nitrocefin (10 to 1000 μM) was added. Absorbance at 486 nm was recorded over the time. Initial 10 min data was used for determining the hydrolysis rate which then fit into Michaelis-Menten equation to obtain $K_M$ of the β-lactamase-E104D with or without BLIP. Inhibition constant, $K_i$, of the BLIP was obtained using **Eq. 7.** In **Eq. 7** $K_{m\,(+BLIP)}$ and $K_M$ are the Michaelis-Menten in the presence and absence of BLIP, respectively.

$$K_{m\,(+BLIP)} = K_m\,(1+([\text{BLIP}]/K_i)) \qquad (7)$$

**Supplemental Information**

**Peptide synthesis**

**The** peptides were synthesized from C- to N-terminus on 200 μmol scale using manual solid phase peptide synthesis. Rink Amide MBHA resin (340 mg, loading: 0.59 mmol/g) was put in a 20 mL syringe with filter, and allowed to swell in NMP for 30 minutes on a shaker. A 400 mM stock solution of HCTU was prepared. A 20% piperidine in NMP solution was used for Fmoc deprotection and a 3/1/1 NMP/$Ac_2O$/pyridine solution was used for capping. All amino acids were dissolved in NMP, creating stock solutions of 200 mM. DIPEA could be used directly out of the bottle. The Fmoc-group was removed using 20% piperidine (2 times 5 minutes on a shaker) followed by an NMP wash (3 times, shake syringe by hand for approximately 30 seconds). Before coupling, amino acids were preactivated (5 minutes) using 4 mL of 200 mM amino acid solution (4 eq.) with 2 ml of 400 mM HCTU (4 eq.). 279 μL of DIPEA (8 eq.) was added and the amino acid was coupled for 30 minutes on a shaker, followed by a NMP wash. Capping was performed using 3/1/1 NMP/$Ac_2O$/Pyridine solution (2 times 5 minutes on a shaker) followed by an NMP wash. The first amino acid (on the C-terminus) was coupled 2 times for 45 minutes. Because the length of the peptide, synthesis was sometimes stopped halfway (just before the capping step), washed 2 times with DCM and dried under vacuum for 30 minutes. The unfinished peptide was stored in the fridge overnight. When synthesis was continued the resin was again swollen in NMP for 30 minutes, followed by a capping step. When the peptide was fully synthesized, an extra deprotection and capping step were performed to protect the N-terminus, yielding an acetylated N-terminus. Then again a NMP wash (3 times) and a DCM wash (2 times) were performed and the peptide (on the resin) was dried under vacuum. Cleavage was accomplished using 5 mL 95/2.5/2.5 TFA/$H_2O$/TIS and shaking for 3 hours on a shaker. The cleaved peptides were transferred to a 50 mL Falcon tube. The cleaved and deprotected peptide was precipitated by adding diethylether (up to 50 mL), shaken on a vortex and stored in a -30 degrees Celsius freezer for at least 2 hours. The ethereal layer was decanted off after centrifugation (2,000 rpm for 10 min), this step was repeated and the remaining diethylether was evaporated by storing the open Falcon tube in a fume hood for 30 minutes. Peptides were dissolved in $H_2O$ (+ 0.1 % TFA) and ACN, starting with a ratio of 95/5 and adding more ACN if not well dissolved. The solutions were filtered through a 0.45 μm filter using PALL Acrodisc syringe filters with supor membrane. For characterization, LC-MS was performed on the filtrate using a LC-MS (Shimadzu SCL-10 AD VP series HPLC coupled to a diode array detector (Finnigan Surveyor PDA Plus detector, Thermo Electron Corporation) and an Ion-Trap S3 (LCQ Fleet, Thermo Scientific)). A gradient of 2-70% of ACN in $H_2O$ (+0.1 % TFA) was used. Purification was performed on prep-RP-HPLC (Shimadzu) using a C18- column using 10 to 50% ACN in $H_2O$ (+0.1 % TFA) with a flow rate of 15 mL/min. All peptides were acetylated at N-terminus and amidated at the C-terminus.

**Coupling of fluorescein to thiol group of the peptide:** 10 mg of the thiol-containing peptide (1 eq.) and TCEP (2 eq.) were dissolved in pH 7.0 HBS buffer (100 mM HEPES + NaCl 100 mM). The pH was adjusted to 7.0 and then fluorescein-5-maleimide (Invitrogen, Cat# F-150) (1.5 eq.) was added and stirred at RT for overnight. The product was purified by prep-RP-HPLC. Analysis and characterization was done with LC-MS. The ESI-MS indicated that part of the product had been converted to a +18 Da derivative, which results from hydrolytic ring opening of a maleimide-derived succinimide group. Since this modification is unlikely to affect the binding properties of the peptides, we did not attempt to separate both species.

**HIV1-p17 short epitope**

WEKIRLR-GGG-C **(SEQ ID NO:10)**
*LC-MS: m/z [M + 2H]2+ Calcd. 658.78 Da Obsd. 658.75 Da; [M]+ Calcd. 1315.55 Da Obsd. 1315.92 Da*

WEKIRLR-GGG-C(fluorescein)
*LC-MS: m/z [M + 2H]2+ Calcd. 872.97 Da Obsd. 872.58 Da; [M]+ Calcd. 1743.92 Da Obsd. 1743.92 Da; And [M+H2O+H]2+ Calcd. 881.47 Da Obsd. 881.33 Da;[M+H20]+ Calcd. 1761.93 Da Obsd. 1761.92 Da*

**HIV1-p17 longer epitope**

ELDRWEKIRLRP **(SEQ ID NO:3)**
*LC-MS: m/z [M + 3H]3+ Calcd. 551.64 Da Obsd. 551.75 Da; [M+2H]2+ Calcd. 826.96 Da Obsd. 826.83 Da; [M+H]+ Calcd. 1652.92 Da Obsd. 1652.92 Da*

ELDRWEKIRLRP-GGG-C **(SEQ ID NO:2)**
*LC-MS: m/z [M + 3H]3+ Calcd. 643.08 Da Obsd. 643.25 Da; [M+H]2+ Calcd. 963.61 Da Obsd. 963.83 Da; [M +H]+ Calcd. 1927.21 Da Obsd. 1927.08 Da*

ELDRWEKIRLRP-GGG-C(fluorescein)
*LC-MS: m/z [M + H]2+ Calcd. 1177.95 Da Obsd. 1177.67 Da; [M +2H]3+ Calcd. 785.53 Da Obsd. 785.58 Da; [M +3H]4+ Calcd. 589.40 Da Obsd. 589.58 Da*

**HA-tag peptide**

YPYDVPDYA **(SEQ ID NO:5)**
*LC-MS: m/z [M + 2H]2+ Calcd. 572.61 Da Obsd. 572.50 Da; [M]+ Calcd. 1143.20 Da Obsd. 1143.75 Da*

YPYDVPDYA-GGG-C **(SEQ ID NO:4)**
*LC-MS: m/z [M + 2H]2+ Calcd. 709.76 Da Obsd. 709.58 Da; [M]+ Calcd. 1417.50 Da Obsd. 1417.75 Da*

YPYDVPDYA-GGG-C(fluorescein)
*LC-MS: m/z [M + 3H]3+ Calcd. 616.30 Da Obsd. 616.00 Da; [M+2H]2+ Calcd. 923.44 Da Obsd. 923.58 Da; [M+H] + Calcd. 1846.88 Da Obsd. 1846.67 Da;*
*And [M+H2O+2H]3+ Calcd. 621.97 Da Obsd. 621.92 Da; [M+H2O+H]2+ Calcd. 932.45 Da Obsd. 932.58 Da*

**DEN1 peptide**

EHKYSWKS-GGG-C **(SEQ ID NO:6)**
*LC-MS: m/z [M + 3H]3+ Calcd. 460.54 Da Obsd. 461.00 Da; [M+2H]2+ Calcd. 690.31 Da Obsd. 690.75 Da; [M+H] + Calcd. 1379.62 Da Obsd. 1379.83 Da;*

EHKYSWKS-GGG-C(fluorescein)

*LC-MS: m/z [M + 4H]4+ Calcd. 452.43 Da Obsd. 452.92 Da; [M+3H]3+ Calcd. 602.90 Da Obsd. 603.33 Da; [M+2H] 2+ Calcd. 903.85 Da Obsd. 904.17 Da;*

*and[M+H2O+4H]4+ Calcd.* 456.93 *Da Obsd. 457.42 Da; [M+H2O+3H]3+ Calcd. 608.90 Da Obsd. 609.33 Da; [M+H2O+2H]2+ Calcd. 912.85 Da Obsd. 913.50 Da;*

**Peptide binding assays using fluorescence polarization**

To determine the affinity of the antibodies for their peptide epitopes, a GGGC sequence was introduced at the C-termini of the epitope sequences. The C-terminal cysteine was used to attach a fluorescein by reacting the cysteine with maleimide-functionalized fluorescein. Binding of antibody to the fluorescently-labeled peptide results in an increase in fluorescence polarization. **Eq. 8** was used to fit the polarization as a function of the concentration of antigen binding domains, yielding the dissociation constant for the interaction. In this analysis it was assumed that binding of peptide to each of the antigen binding domains was independent.

$$A = A_f + \left(A_b - A_f\right) * \frac{([P]+K_d+[Ab])-\sqrt{([P]+K_d+[Ab])^2-4[P][Ab]}}{2[P]} \quad (8)$$

In **Eq. 8,** A is the measured polarization, $A_f$ is the polarization of the free peptide, $A_b$ is polarization value of the bound peptide, [P] is the peptide concentration and [Ab] is the concentration of antigen binding domains.

**FIG. 10** shows titration of 10 nM (WEKIRLR-GGG-C(fluorescein)) with anti-HIV1-p17 antibody monitored using fluorescence polarization. The data were fit to **Eq. 8** yielding a $K_d$ of 3.3 ± 0.2 μM.

**Affinity of the long HIV1-p17 epitope (ELDRWEKIRLRP)**

First we determined the affinity of the anti HIV1-p17 antibody by titration of the antibody to 10 nM of fluorescein-labeled peptide (ELDRWEKIRLRP-GGG-C(fluorescein)). This titration yielded a $K_d$ of 24±3 nM **(FIG. 11).** To test whether the fluorescein label influences the interaction with the antibody, we also performed a competition assay in which a fixed concentration of antibody and fluorescently-labeled peptide was titrated with non- fluorescent peptide (ELDRWEKIRLRP; **SEQ ID NO:3).** The competitive titration was fit to **Eq. 9** to yield an EC50 **(FIG. 12).** This EC50 was subsequently used to calculate the affinity of the antibody for the non-labeled peptide using **Eq. 10,** yielding a *K*d1 value of 42±1 nM.

$$A = A_i + \frac{(A_i - A_f)}{(1+10^{(log[P]-logEC50)})} \quad (9)$$

$$logEC50 = \log(10^{Kd_1} * \left(\frac{1+[P_{fl}]}{Kd_2}\right)) \quad (10)$$

with $Kd_1$ is the dissociation constant for the non-labeled peptide, $Kd_2$ the dissociation constant for the fluorescently labeled peptide, [P] the concentration of the unlabeled peptide, and $[P_{fl}]$ the concentration of the fluorescent peptide.

**Affinity of HA-tag epitope antibody interaction**

First we determined the affinity of the HA-tag antibody by titration of the antibody to 2 nM of fluorescein-labeled peptide (YPYDVPDYA-GGG-C(fluorescein)). This titration showed tight binding and yielded a $K_d$ = 0.58±0.22 nM **(FIG. 13).** To test whether the fluorescein label influences the interaction with the antibody, we also performed a competition assay in which a fixed concentration of antibody and fluorescently-labeled peptide was titrated with non-fluorescent peptide (YPYDVPDYA; **SEQ ID NO:5**). The competitive titration was fit to **Eq. 9** to yield an EC50 **(FIG. 14).** This EC50 was subsequently used to calculate the affinity of the antibody for the non-labeled peptide using **Eq. 10,** yielding a $K_{d1}$ value of 4.5±1 nM.

**Affinity of epitope-antibody interaction of Dengue type I specific antibody**

**FIG. 15** shows titration of 10 nM (EHKYSWKS-GGG-C(fluorescein)) with anti-Dengue-1 antibody monitored using fluorescence polarization. The data were fit to **Eq. 8** yielding a $K_d$ of 70 ± 13 nM.

**Characterization of Abs-3 variants with single point mutations in lactamase or BLIP**

**FIG. 16** shows enzymatic activity of **Abs-3** mutants (0.3 nM) in the presence and absence of 200 nM of the anti-HIV1-p17 antibody. Antibody and sensor were incubated for 1 h at RT. Then 50 μM of nitrocefin was added and the measurement was started immediately. The assay was performed in pH 7.0 phosphate buffer (50 mM) that contains NaCl (100 mM) and BSA (1 mg/mL).

**Determination of inhibition constants of BLIP mutants -lactamase-E104D**

5 μM BLIP protein was added to 100 pM of beta-lactamase-E104D and incubated for 2 h at 30 degrees Celsius. Aliquots of enzyme alone or enzymeinhibitor complex were pipetted into a 96-well plate. To these samples different concentrations of nitrocefin (10 to 1000 μM) were added. The hydrolysis rate was determined by monitoring the increase in absorbance at 486 nm for 10 minutes. Non-linear least square fitting of the hydrolysis rates as a function of nitrocefin concentration using the Michaelis-Menten equation was used to determine $K_M$ values in the absence and presence of BLIP. The *Ki* was calculated using **Eq. 7,** which represent the relation between the 2 $K_M$ values, the inhibitor concentration and $K_i$ for a competitive inhibitor.

## Claims

**1.** An antibody detection method, comprising:

using a biosensor for detecting an antibody,

wherein said biosensor comprises an enzyme covalently linked to an inhibitor protein via a peptide linker having two epitopes at the ends of said peptide linker, and wherein said biosensor is defined by two equilibrium constants, $K_{closed\text{-}open,1}$ and $K_{closed\text{-}open,2}$ that describe the equilibrium between a closed and an open state of said biosensor in the absence and presence, respectively, of said antibody according to:

$$K_{closed\text{-}open,1} = K_{d(EI)}/C_{eff,(EI)},$$

and

$$K_{closed\text{-}open,2} = 0.5 * K_{d,(EI)}/K_{d(AP)} * C_{eff,(AP)}/C_{eff(EI)}$$

wherein:

$K_{d(Ap)}$ is an intermolecular dissociation constant of a monovalent binding of said antibody and said epitope,
$K_{d(EI)}$ is an intermolecular dissociation constant of the binding of said enzyme and said inhibitor protein,
$C_{eff(EI)}$ is an effective concentration of said inhibitor protein in proximity of said enzyme, and
$C_{eff(AP)}$ is an effective concentration of a free epitope in proximity of the remaining antigen-binding domain of said antibody; and

wherein said $K_{closed\text{-}open,1}$ is smaller than said $K_{closed\text{-}open,2}$.

**2.** The method as set forth in claim 1, wherein said $K_{closed\text{-}open,1}$ is less than 3.

**3.** The method as set forth in claim 1, wherein said $K_{closed\text{-}open,1}$ is larger than 0 and less than 0.2.

**4.** The method as set forth in claim 1, wherein said $K_{closed\text{-}open,2}$ is greater than 0.2.

**5.** The method as set forth in claim 1, wherein said $K_{closed\text{-}open,2}$ is larger than 0.2 and less than $10^6$.

**6.** An *in vitro* antibody-detecting method, comprising:

(a) contacting a sample with a biosensor; said biosensor comprising:

(i) a reporter enzyme;
(ii) an inhibitor domain having affinity for said reporter enzyme;
(iii) at least two epitopes, each epitope having affinity for said antibody; and
(iv) a linker;

(b) determining the activity of said reporter enzyme in the presence of a sample; and
(c) attributing the activity of said reporter enzyme in the presence of said sample to the quantitative or qualitative presence or absence of an antibody,

wherein, in the absence of said antibody, said biosensor is in a closed, inactive state in which at least some of said reporter enzyme forms an intramolecular complex with said inhibitor domain, and
wherein a bivalent binding between two antigen binding domains present in said antibody and said at least two epitopes present at the ends of said linker between said reporter enzyme and said inhibitor domain in said biosensor changes the equilibrium between the closed (inactive) and an open (active) state of said biosensor;
wherein the equilibrium between the closed (inactive) and the open (active) state of said biosensor in the absence of said antibody is smaller than the equilibrium between the closed (inactive) and the open (active) state of said biosensor in the presence of said antibody; such that the amount of said reporter enzyme that forms an intramolecular complex with said inhibitor domain is decreased.

**7.** The method as set forth in claim 6, wherein said reporter enzyme is beta-lactamase or a fragment thereof.

**8.** The method as set forth in claim 6, wherein said inhibitor domain is a beta-lactamase inhibitor protein or a fragment thereof.

**9.** The method as set forth in claim 6, wherein, in the closed state, said at least some of said reporter enzyme forms an intramolecular complex with said inhibitor domain is at least 30% of said reporter enzyme forms an intramolecular complex with said inhibitor domain.

**10.** The method as set forth in claim 6, wherein, in the closed state, said at least some of said reporter enzyme forms an intramolecular complex with said inhibitor domain is at least 50% of said reporter enzyme forms an intramolecular complex with said inhibitor domain.

**11.** The method as set forth in claim 6, wherein, in the closed state, said at least some of said reporter enzyme forms an intramolecular complex with said inhibitor domain is at least 80% of said reporter enzyme forms an intramolecular complex with said inhibitor domain.

## Patentansprüche

**1.** Antikörper-Nachweisverfahren, das Folgendes umfasst:

Verwendung eines Biosensors zum Nachweis eines Antikörpers,

wobei der Biosensor ein Enzym umfasst, das mit einem Inhibitorprotein über einen Peptid-Linker, der zwei Epitope an den Enden des Peptid-Linkers aufweist, kovalent verknüpft ist, und
wobei der Biosensor durch zwei Gleichgewichtskonstanten, $K_{closed\text{-}open,1}$ und $K_{closed\text{-}open,2}$, definiert ist, die das Gleichgewicht zwischen einem geschlossenen und einem offenen Zustand des Biosensors in Abwesenheit bzw. Gegenwart des Antikörpers beschreiben entsprechend:

$$K_{closed\text{-}open,1} = K_{d(EI)}/C_{eff,(EI)}$$

und

$$K_{closed\text{-}open,2} = 0{,}5 * K_{d,(EI)}/K_{d(AP)} * C_{eff,(AP)}/C_{eff(EI)} \quad ,$$

wobei:

$K_{d(AP)}$ eine intermolekulare Dissoziationskonstante einer monovalenten Bindung des Antikörpers und des Epitops ist,
$K_{d(EI)}$ eine intermolekulare Dissoziationskonstante der Bindung des Enzyms und des Inhibitorproteins ist,
$C_{eff(EI)}$ eine wirksame Konzentration des Inhibitorproteins in der Nähe des Enzyms ist und
$C_{eff(AP)}$ eine wirksame Konzentration eines freien Epitops in der Nähe
der verbleibenden Antigenbindungsdomäne des Antikörpers ist; und wobei die $K_{closed\text{-}open,1}$ kleiner ist als die $K_{closed\text{-}open,2}$.

**2.** Verfahren nach Anspruch 1, wobei die $K_{closed\text{-}open,1}$ kleiner als 3 ist.

**3.** Verfahren nach Anspruch 1, wobei die $K_{closed\text{-}open,1}$ größer als 0 und kleiner als 0,2 ist.

**4.** Verfahren nach Anspruch 1, wobei die $K_{closed\text{-}open,2}$ größer als 0,2 ist.

**5.** Verfahren nach Anspruch 1, wobei die $K_{closed\text{-}open,2}$ größer als 0,2 und kleiner als $10^6$ ist.

**6.** In-vitro-Antikörper-Nachweisverfahren, das Folgendes umfasst:

(a) Inkontaktbringen einer Probe mit einem Biosensor; wobei der Biosensor Folgendes umfasst:

(i) ein Reporterenzym;
(ii) eine Inhibitordomäne, die eine Affnität für das Reporterenzym aufweist;
(iii) mindestens zwei Epitope, wobei jedes Epitop eine Affinität für den Antikörper aufweist; und
(iv) einen Linker;

(b) Bestimmen der Aktivität des Reporterenzyms in Gegenwart einer Probe; und
(c) Zuordnen der Aktivität des Reporterenzyms in Gegenwart der Probe der quantitativen oder qualitativen Gegenwart oder Abwesenheit eines Antikörpers, wobei in Abwesenheit des Antikörpers der Biosensor in einem geschlossenen, inaktiven Zustand vorliegt, in dem mindestens einiges des Reporterenzyms einen intramolekularen Komplex mit der Inhibitordomäne bildet, und

wobei eine bivalente Bindung zwischen zwei Antigenbindungsdomänen, die in dem Antikörper vorliegen, und den mindestens zwei Epitopen, die an den Enden des Linkers zwischen dem Reporterenzym und der Inhibitordomäne in dem Biosensor vorliegen, das Gleichgewicht zwischen dem geschlossenen (inaktiven) und einem offenen (aktiven) Zustand des Biosensors verändert;
wobei das Gleichgewicht zwischen dem geschlossenen (inaktiven) und dem offenen (aktiven) Zustand des Biosensors in Abwesenheit des Antikörpers kleiner ist als das Gleichgewicht zwischen dem geschlossenen (inaktiven) und dem offenen (aktiven) Zustand des Biosensors in Gegenwart des Antikörpers, sodass die Menge des Reporterenzyms, das einen intramolekularen Komplex mit der Inhibitordomäne bildet, reduziert ist.

**7.** Verfahren nach Anspruch 6, wobei das Reporterenzym Beta-Lactamase oder ein Fragment davon ist.

**8.** Verfahren nach Anspruch 6, wobei die Inhibitordomäne ein Beta-Lactamase-Inhibitorprotein oder ein Fragment davon ist.

**9.** Verfahren nach Anspruch 6, wobei in dem geschlossenen Zustand das mindestens einige des Reporterenzyms, das einen intramolekularen Komplex mit der Inhibitordomäne bildet, mindestens 30 % des Reporterenzyms beträgt, das einen intramolekularen Komplex mit der Inhibitordomäne bildet.

**10.** Verfahren nach Anspruch 6, wobei in dem geschlossenen Zustand das mindestens einige des Reporterenzyms, das einen intramolekularen Komplex mit der Inhibitordomäne bildet, mindestens 50 % des Reporterenzyms beträgt, das einen intramolekularen Komplex mit der Inhibitordomäne bildet.

**11.** Verfahren nach Anspruch 6, wobei in dem geschlossenen Zustand das mindestens einige des Reporterenzyms, das einen intramolekularen Komplex mit der Inhibitordomäne bildet, mindestens 80 % des Reporterenzyms beträgt, das einen intramolekularen Komplex mit der Inhibitordomäne bildet.

## Revendications

1. Méthode de détection d'un anticorps comprenant :

l'utilisation d'un biocapteur pour détecter un anticorps,

où ledit biocapteur comprend une enzyme liée de manière covalente à une protéine inhibitrice par l'intermédiaire d'un coupleur peptidique ayant deux épitopes aux extrémités dudit coupleur peptidique et
où ledit biocapteur est défini par deux constantes d'équilibre, $K_{\text{fefmé-ouvert},1}$ et $K_{\text{fermé-ouvert},2}$, qui décrivent l'équilibre entre un état fermé et un état ouvert dudit biocapteur en l'absence et en la présence, respectivement, dudit anticorps conformément à ce qui suit :

$$K_{\text{fermé-ouvert},1} = K_{d(EI)}/C_{\text{eff},(EI)}$$

et

$$K_{\text{fermé-ouvert},2} = 0{,}5 * K_{d,(EI)}/K_{d(AP)} * C_{\text{eff},(AP)}/C_{\text{eff}(EI)}$$

où :

$K_{d(Ap)}$ est une constante de dissociation intermoléculaire d'une liaison monovalente entre ledit anticorps et ledit épitope,
$K_{d(EI)}$ est une constante de dissociation intermoléculaire de la liaison entre ladite enzyme et ladite protéine inhibitrice,
$C_{\text{eff}(EI)}$ est une concentration efficace de ladite protéine inhibitrice à proximité de ladite enzyme et
$C_{\text{eff}(AP)}$ est une concentration efficace d'un épitope libre à proximité du domaine de liaison à l'antigène restant dudit anticorps ; et

où ladite $K_{\text{fermé-ouvert},1}$ est plus basse que ladite $K_{\text{fermé-ouvert},2}$.

2. Méthode selon la revendication 1, où ladite $K_{\text{fermé-ouvert},1}$ est inférieure à 3.

3. Méthode selon la revendication 1, où ladite $K_{\text{fermé-ouvert},1}$ est supérieure à 0 et inférieure à 0,2.

4. Méthode selon la revendication 1, où ladite $K_{\text{fermé-ouvert},2}$ est supérieure à 0,2.

5. Méthode selon la revendication 1, où ladite $K_{\text{fermé-ouvert},2}$ est supérieure à 0,2 et inférieure à $10^6$.

6. Méthode de détection d'un anticorps *in vitro* comprenant :

(a) la mise en contact d'un échantillon avec un biocapteur ; ledit biocapteur comprenant :

(i) une enzyme rapporteur ;
(ii) un domaine inhibiteur ayant une affinité pour ladite enzyme rapporteur ;
(iii) au moins deux épitopes, chaque épitope ayant une affinité pour ledit anticorps ; et
(iv) un coupleur ;

(b) la détermination de l'activité de ladite enzyme rapporteur en la présence d'un échantillon ; et
(c) l'attribution, en la présence dudit échantillon, de l'activité de ladite enzyme rapporteur à la présence ou à l'absence, quantitative ou qualitative, d'un anticorps,

où, en l'absence dudit anticorps, ledit biocapteur est dans un état inactif fermé dans lequel au moins une partie de ladite enzyme rapporteur forme un complexe intramoléculaire avec ledit domaine inhibiteur et
où une liaison divalente entre deux domaines de liaison à un antigène présents dans ledit anticorps et lesdits au moins deux épitopes présents aux extrémités dudit coupleur entre ladite enzyme rapporteur et ledit domaine inhibiteur dudit biocapteur modifie l'équilibre entre l'état fermé (inactif) et un état ouvert (actif) dudit biocapteur ;
où l'équilibre entre l'état fermé (inactif) et l'état ouvert (actif) dudit biocapteur en l'absence dudit anticorps est plus bas que l'équilibre entre l'état fermé (inactif) et l'état ouvert (actif) dudit biocapteur en la présence dudit anticorps ;
si bien que la quantité de ladite enzyme rapporteur qui forme un complexe intramoléculaire avec ledit domaine inhibiteur est diminuée.

**7.** Méthode selon la revendication 6, où ladite enzyme rapporteur est une bêta-lactamase ou un fragment de celle-ci.

**8.** Méthode selon la revendication 6, où ledit domaine inhibiteur est une protéine inhibitrice de la bêta-lactamase ou un fragment de celle-ci.

**9.** Méthode selon la revendication 6 où, à l'état fermé, ladite au moins une certaine proportion de ladite enzyme rapporteur formant un complexe intramoléculaire avec ledit domaine inhibiteur représente au moins 30 % de ladite enzyme rapporteur formant un complexe intramoléculaire avec ledit domaine inhibiteur.

**10.** Méthode selon la revendication 6, où, à l'état fermé, ladite au moins une certaine proportion de ladite enzyme rapporteur formant un complexe intramoléculaire avec ledit domaine inhibiteur représente au moins 50 % de ladite enzyme rapporteur formant un complexe intramoléculaire avec ledit domaine inhibiteur.

**11.** Méthode selon la revendication 6, où, à l'état fermé, ladite au moins une certaine proportion de ladite enzyme rapporteur formant un complexe intramoléculaire avec ledit domaine inhibiteur représente au moins 80 % de ladite enzyme rapporteur formant un complexe intramoléculaire avec ledit domaine inhibiteur.

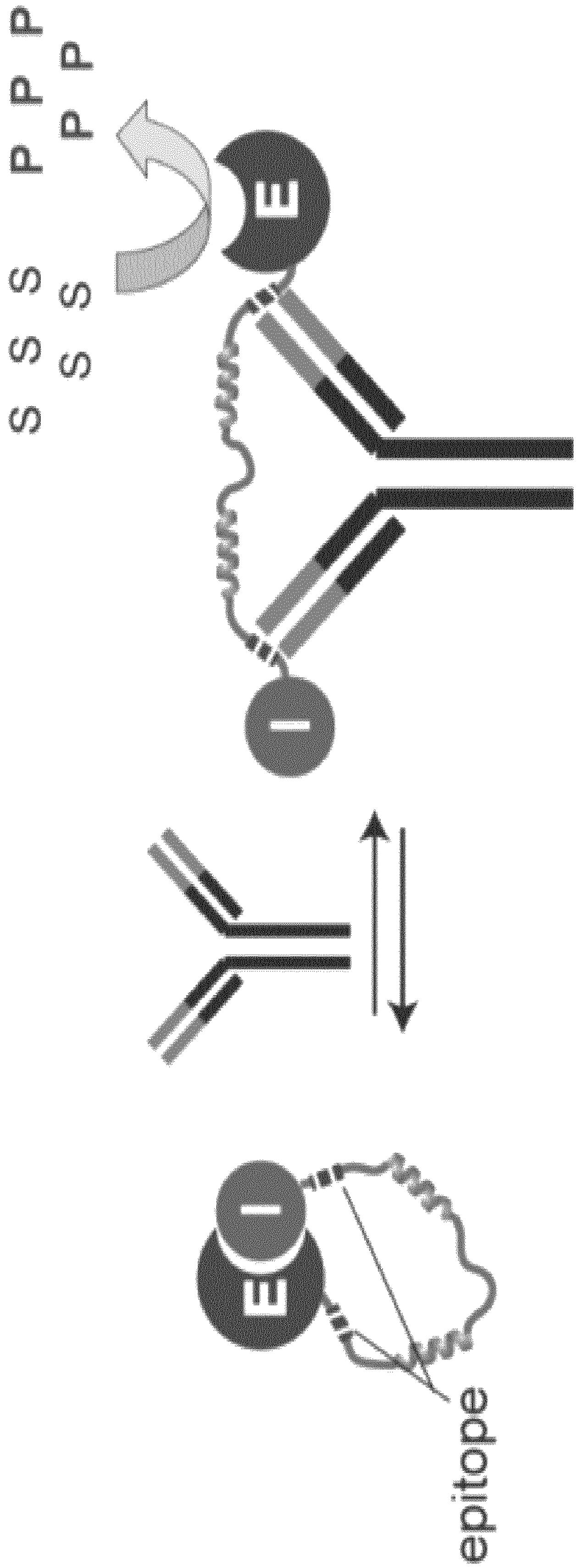


Fig. 1

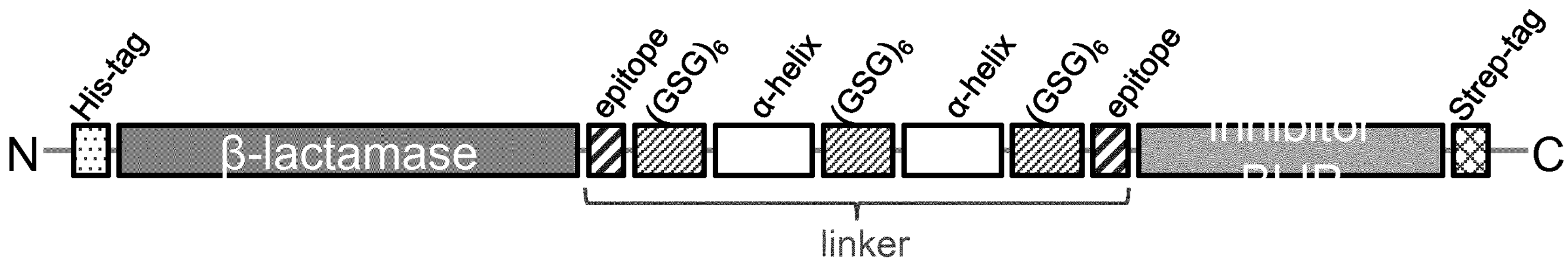


| Abs variant | inhibitor domain | epitope (E) |
|---|---|---|
| Abs-1 | RRGHYY | WEKIRLR (E1) |
| Abs-2 | BLIP | WEKIRLR (E1) |
| Abs-3 | BLIP | ELDRWEKIRLR (E2) |
| Abs-4 | BLIP | YPYDVPDYA (E3) |

**Fig. 2A**

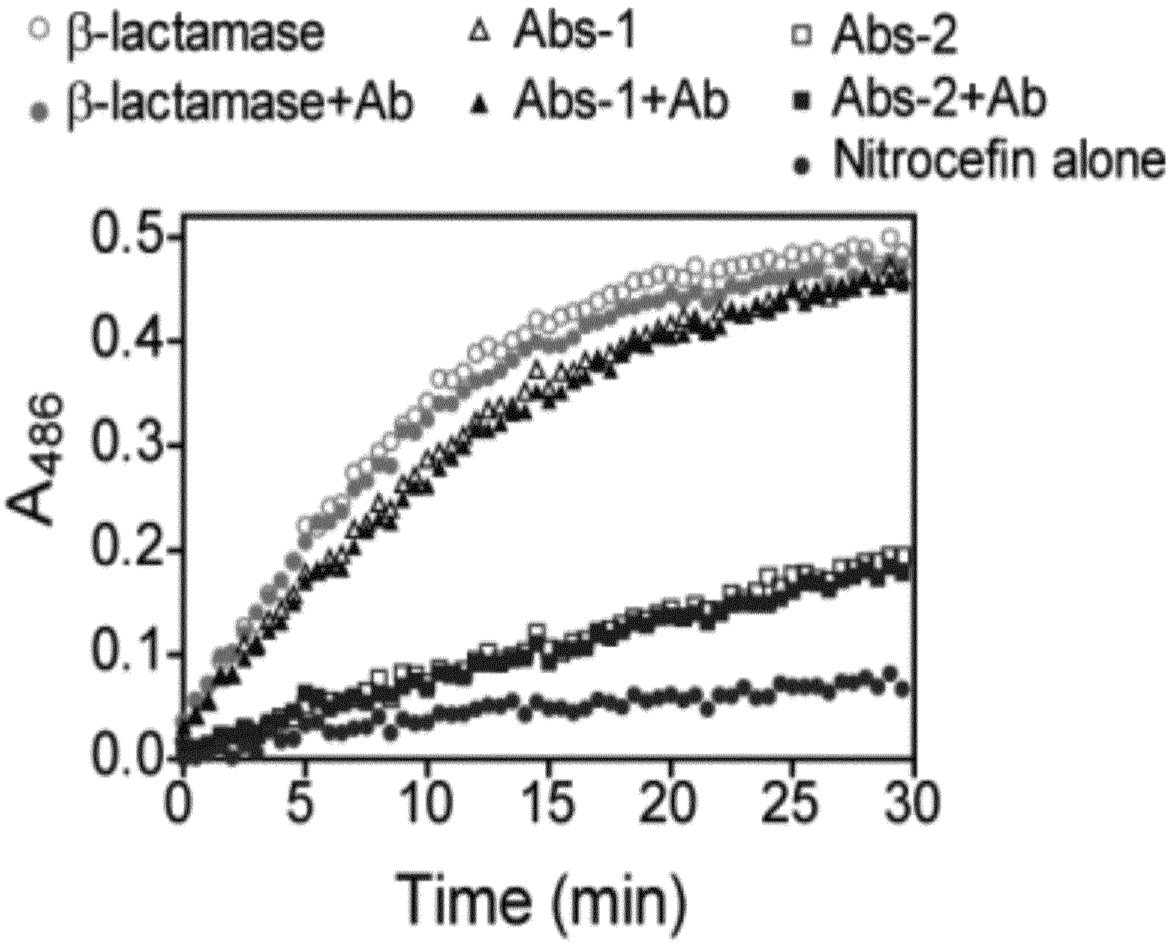
β-lactamase
β-lactamase+Ab
Abs-1
Abs-1+Ab
Abs-2
Abs-2+Ab
Nitrocefin alone
A486
0.0
0.1
0.2
0.3
0.4
0.5
0
5
10
15
20
25
30
Time (min)

Fig. 2B

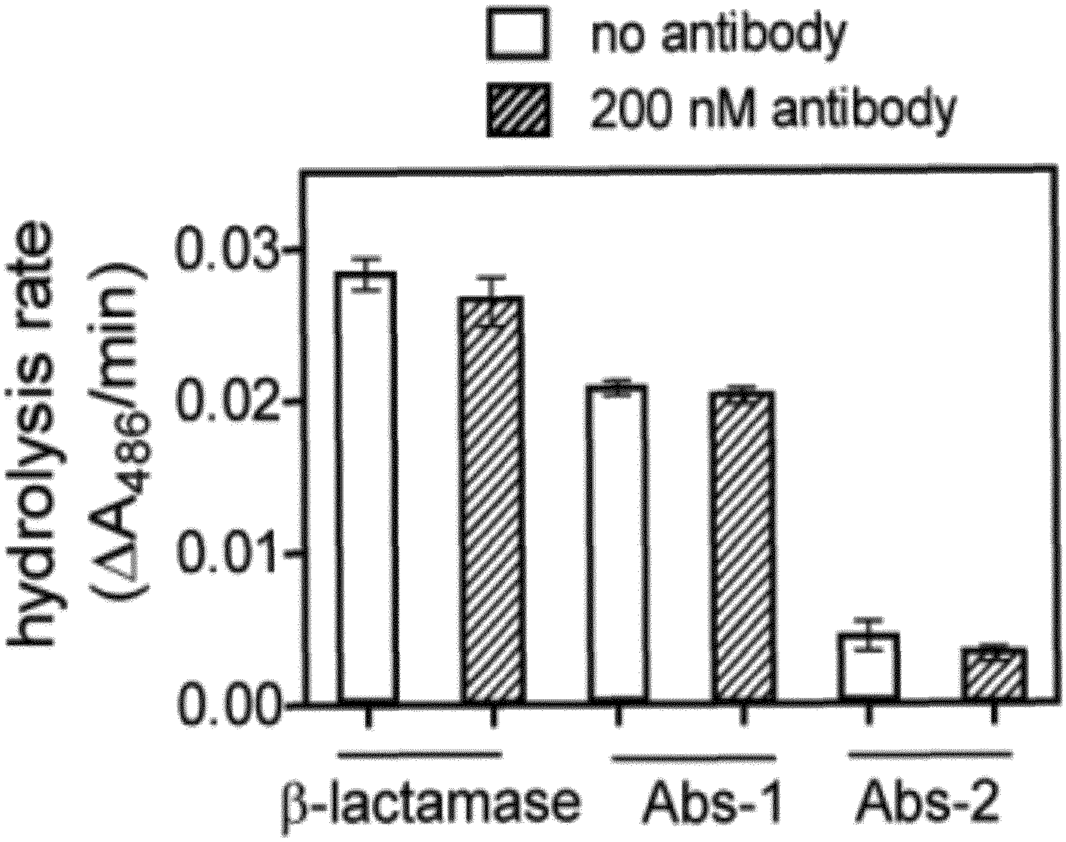
no antibody
200 nM antibody
hydrolysis rate
(ΔA486/min)
0.03
0.02
0.01
0.00
β-lactamase
Abs-1
Abs-2

**Fig. 2C**

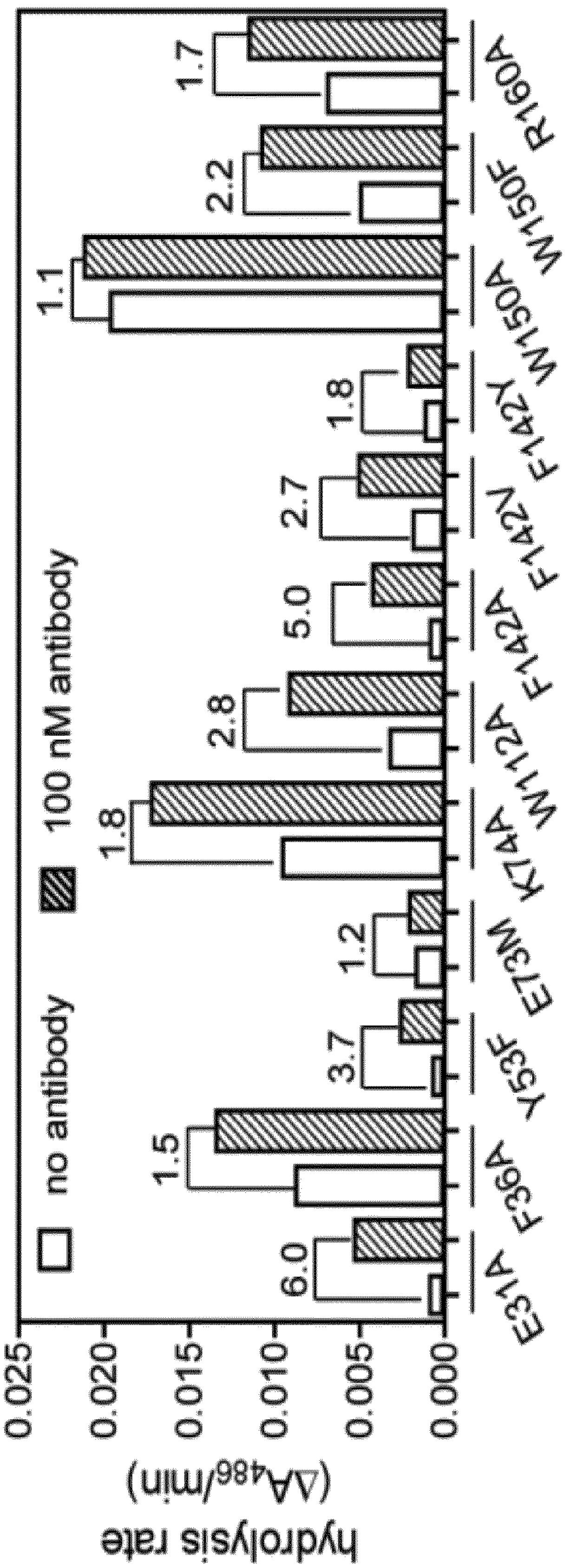
hydrolysis rate
(ΔA486/min)
0.025
0.020
0.015
0.010
0.005
0.000
no antibody
100 nM antibody
6.0
1.5
3.7
1.2
1.8
2.8
5.0
2.7
1.8
1.1
2.2
1.7
E31A
F36A
Y53F
E73M
K74A
W112A
F142A
F142V
F142Y
W150A
W150F
R160A

Fig. 3

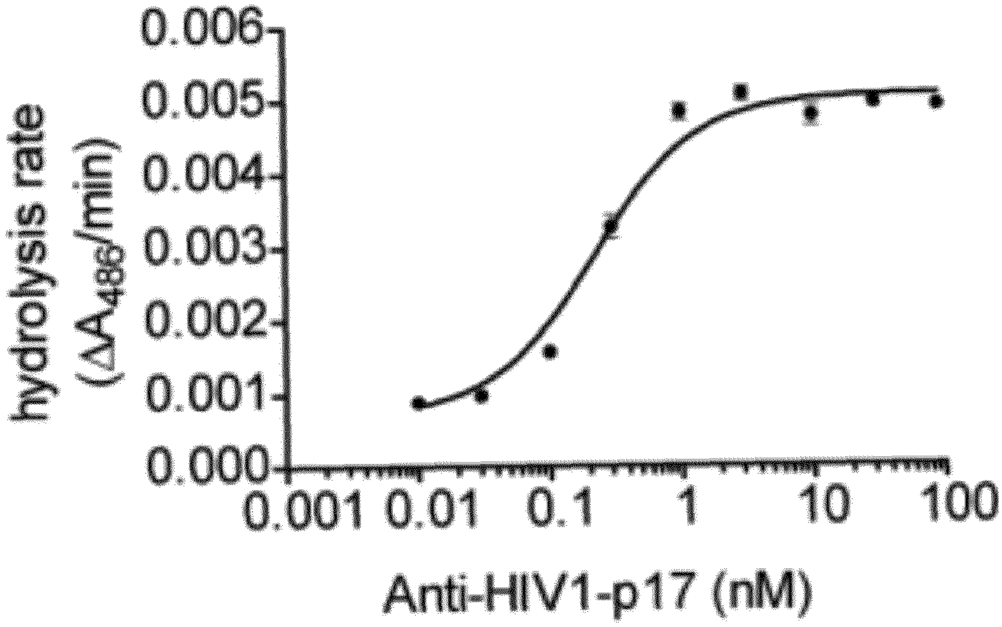
0.006
0.005
0.004
0.003
0.002
0.001
0.000
hydrolysis rate
($\Delta A_{486}$/min)
0.001
0.01
0.1
1
10
100
Anti-HIV1-p17 (nM)

**Fig. 4A**

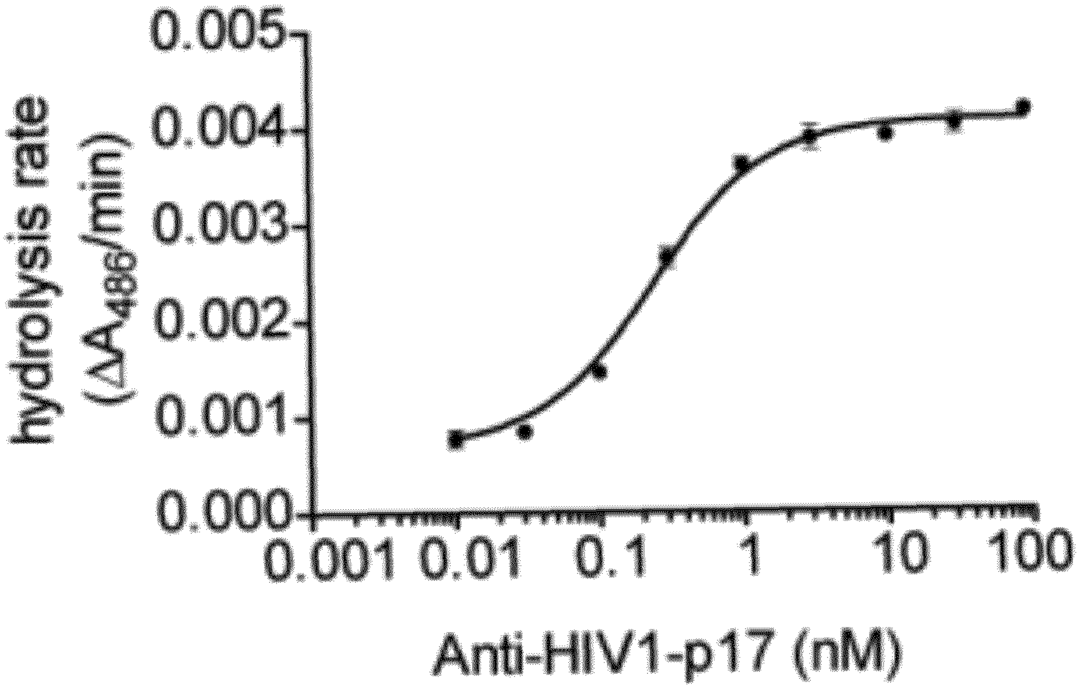
0.005
0.004
0.003
0.002
0.001
0.000
hydrolysis rate
($\Delta A_{486}$/min)
0.001
0.01
0.1
1
10
100
Anti-HIV1-p17 (nM)

**Fig. 4B**

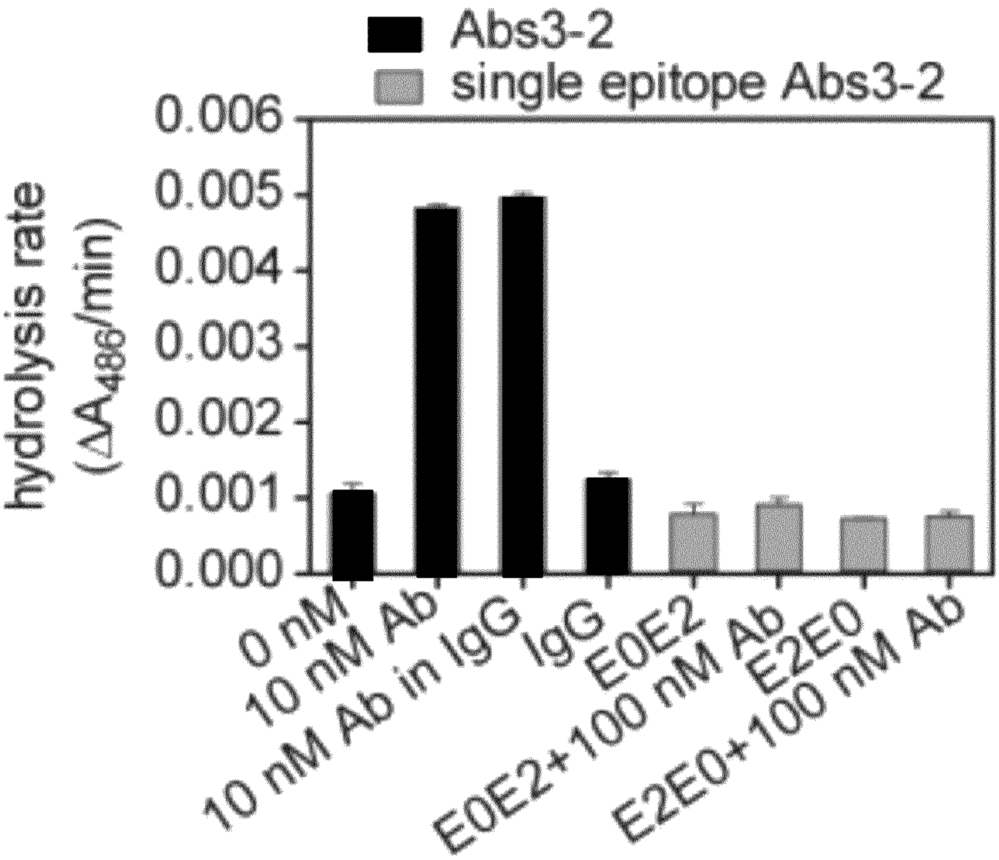

Abs3-2
single epitope Abs3-2
hydrolysis rate ($\Delta A_{486}$/min)
0.006
0.005
0.004
0.003
0.002
0.001
0.000
0 nM
10 nM Ab
10 nM Ab in IgG
IgG
E0E2
E0E2+100 nM Ab
E2E0
E2E0+100 nM Ab


**Fig. 4C**

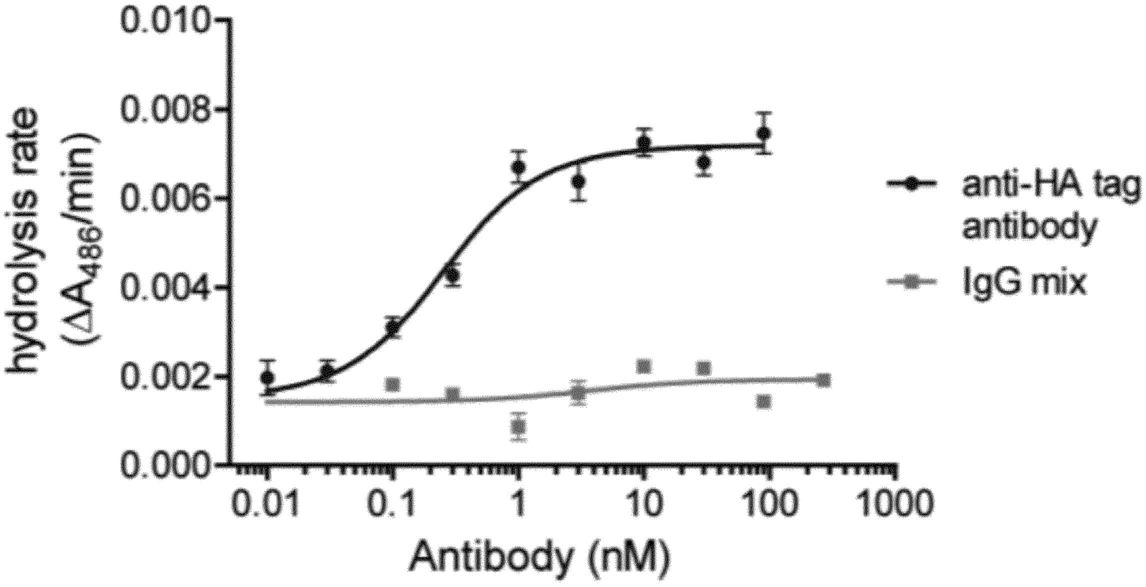
0.010
0.008
0.006
0.004
0.002
0.000
hydrolysis rate
(ΔA486/min)
0.01
0.1
1
10
100
1000
Antibody (nM)
anti-HA tag
antibody
IgG mix

Fig. 5

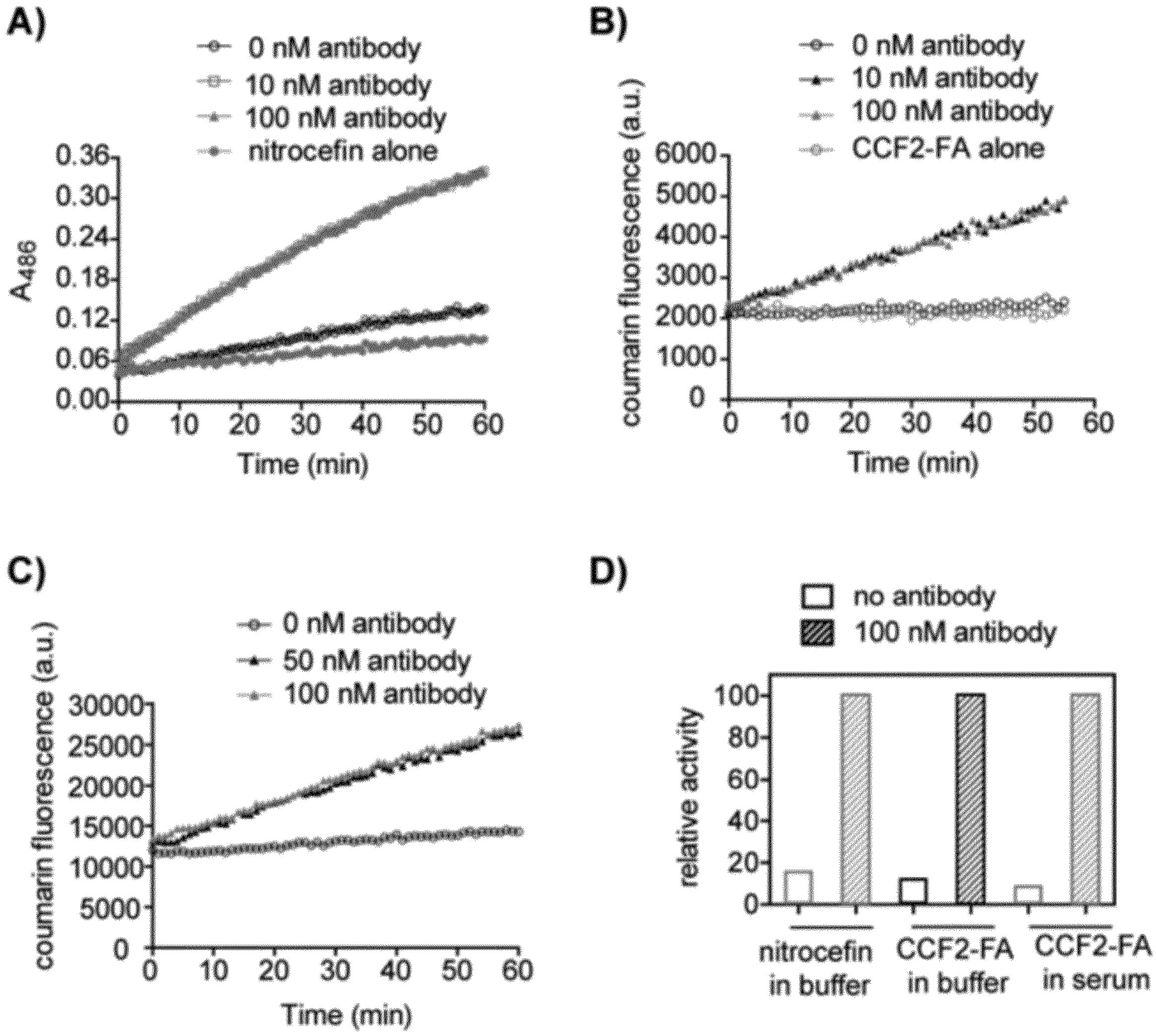
A)
0 nM antibody
10 nM antibody
100 nM antibody
nitrocefin alone
A486
0.36
0.30
0.24
0.18
0.12
0.06
0.00
0 10 20 30 40 50 60
Time (min)
B)
0 nM antibody
10 nM antibody
100 nM antibody
CCF2-FA alone
coumarin fluorescence (a.u.)
6000
5000
4000
3000
2000
1000
0
0 10 20 30 40 50 60
Time (min)
C)
0 nM antibody
50 nM antibody
100 nM antibody
coumarin fluorescence (a.u.)
30000
25000
20000
15000
10000
5000
0
0 10 20 30 40 50 60
Time (min)
D)
no antibody
100 nM antibody
relative activity
100
80
60
40
20
0
nitrocefin in buffer
CCF2-FA in buffer
CCF2-FA in serum

Fig. 6

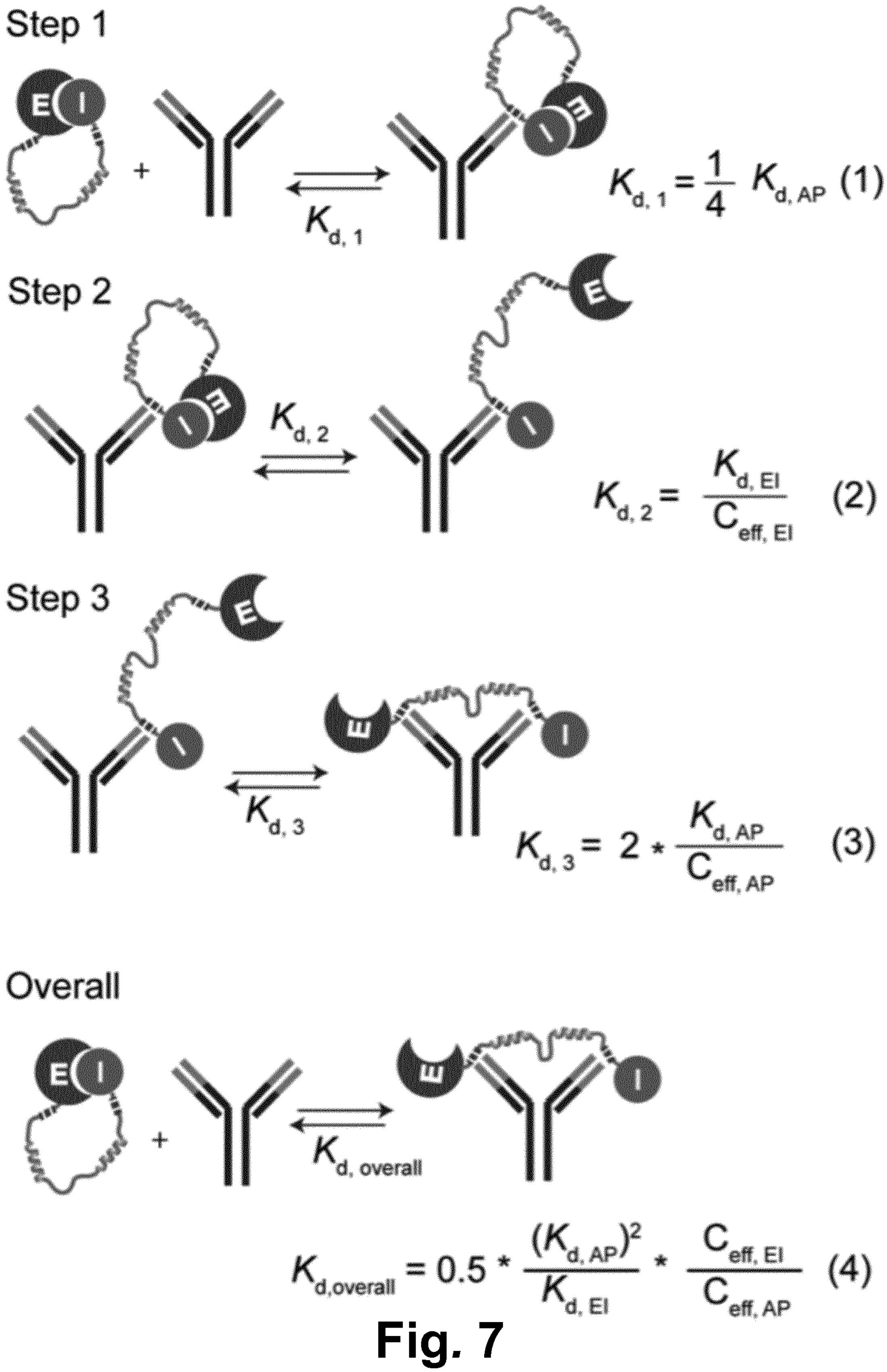
Step 1
E
I
+
$K_{d,1}$
$K_{d,1} = \frac{1}{4} K_{d,AP}$ (1)
Step 2
$K_{d,2}$
$K_{d,2} = \frac{K_{d,EI}}{C_{eff,EI}}$ (2)
Step 3
$K_{d,3}$
$K_{d,3} = 2 * \frac{K_{d,AP}}{C_{eff,AP}}$ (3)
Overall
+
$K_{d,overall}$
$K_{d,overall} = 0.5 * \frac{(K_{d,AP})^2}{K_{d,EI}} * \frac{C_{eff,EI}}{C_{eff,AP}}$ (4)

**Fig. 7**

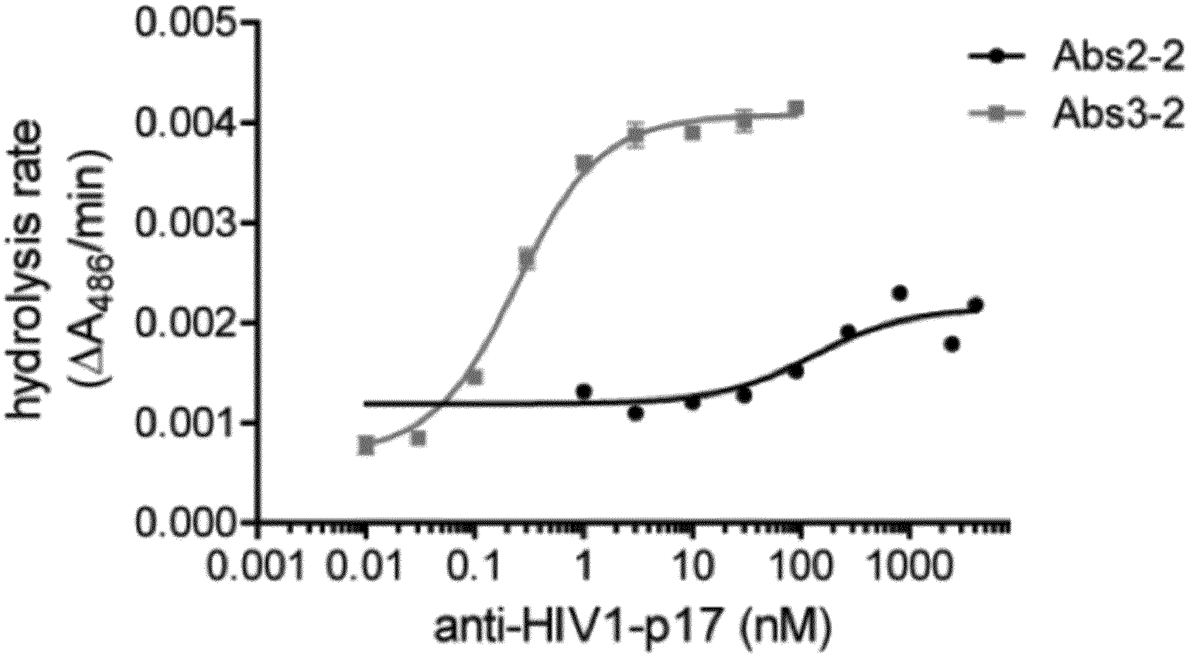

0.005
0.004
0.003
0.002
0.001
0.000
hydrolysis rate
(ΔA486/min)
0.001
0.01
0.1
1
10
100
1000
anti-HIV1-p17 (nM)
Abs2-2
Abs3-2


**Fig. 8**

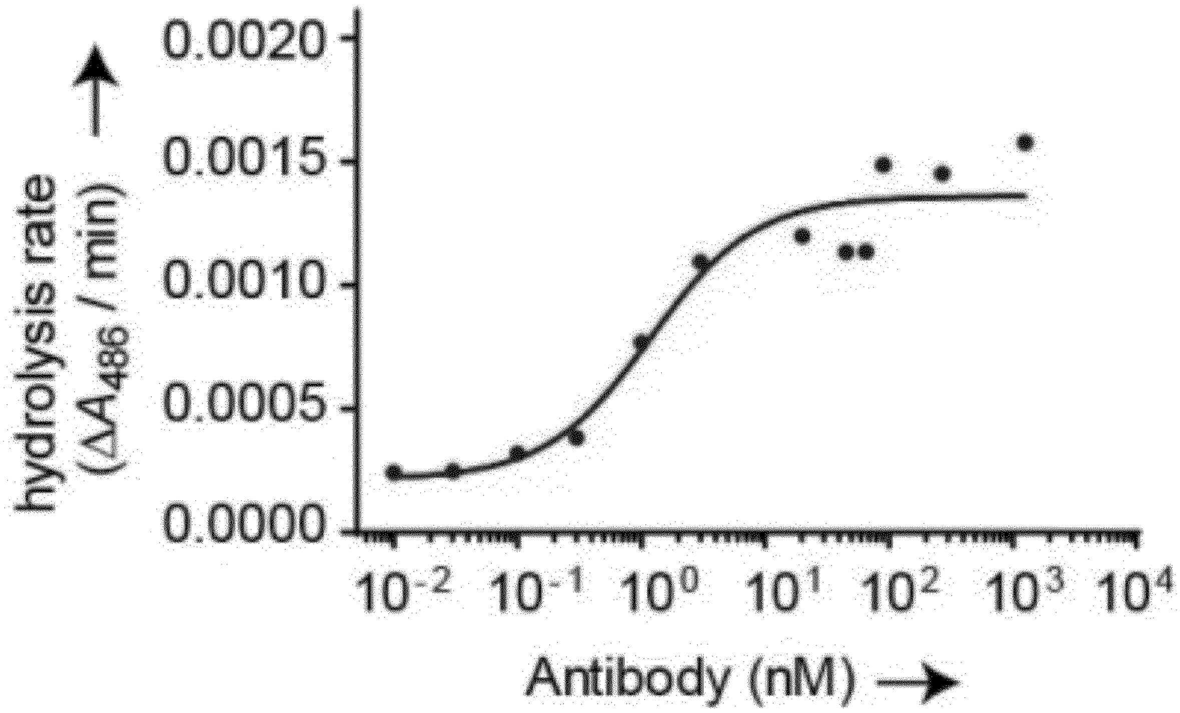
0.0020
0.0015
0.0010
0.0005
0.0000
hydrolysis rate
(ΔA486 / min)
10-2 10-1 100 101 102 103 104
Antibody (nM)

**Fig. 9**

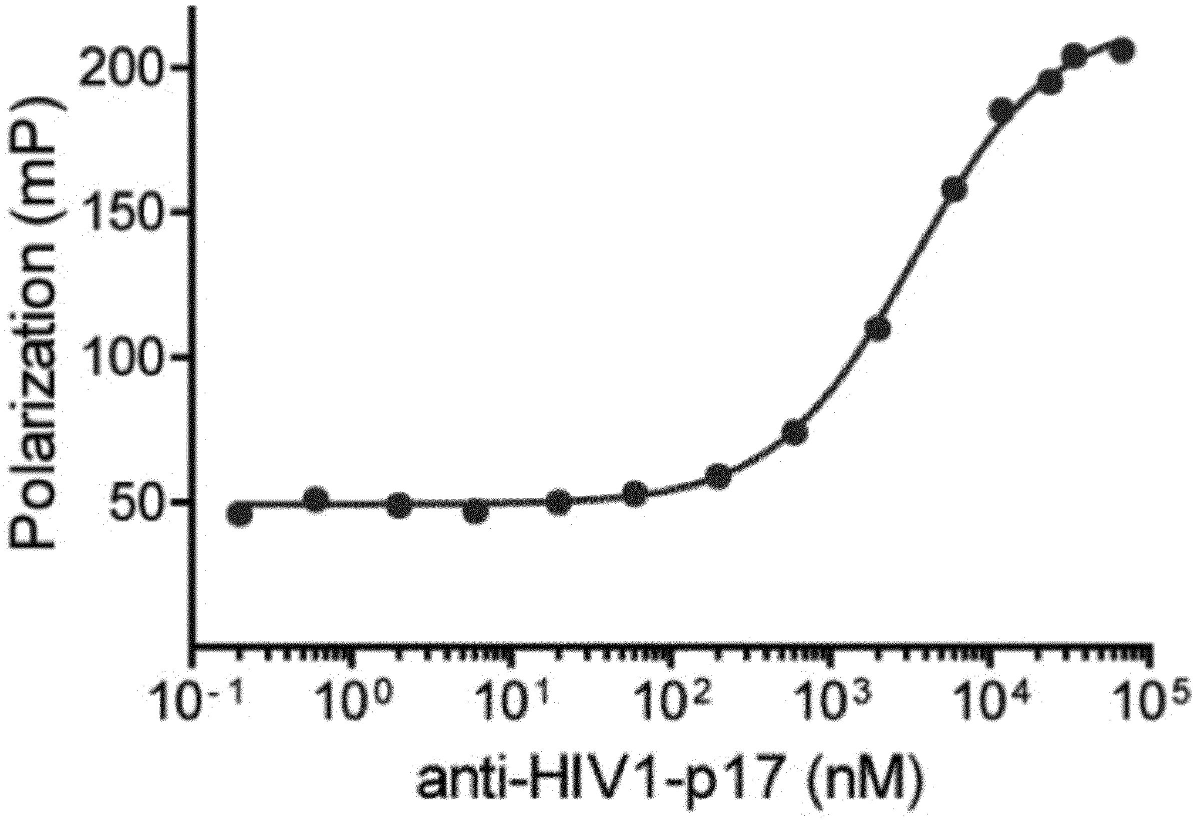
Polarization (mP)
200
150
100
50
10-1
10⁰
10¹
10²
10³
10⁴
10⁵
anti-HIV1-p17 (nM)

**Fig. 10**

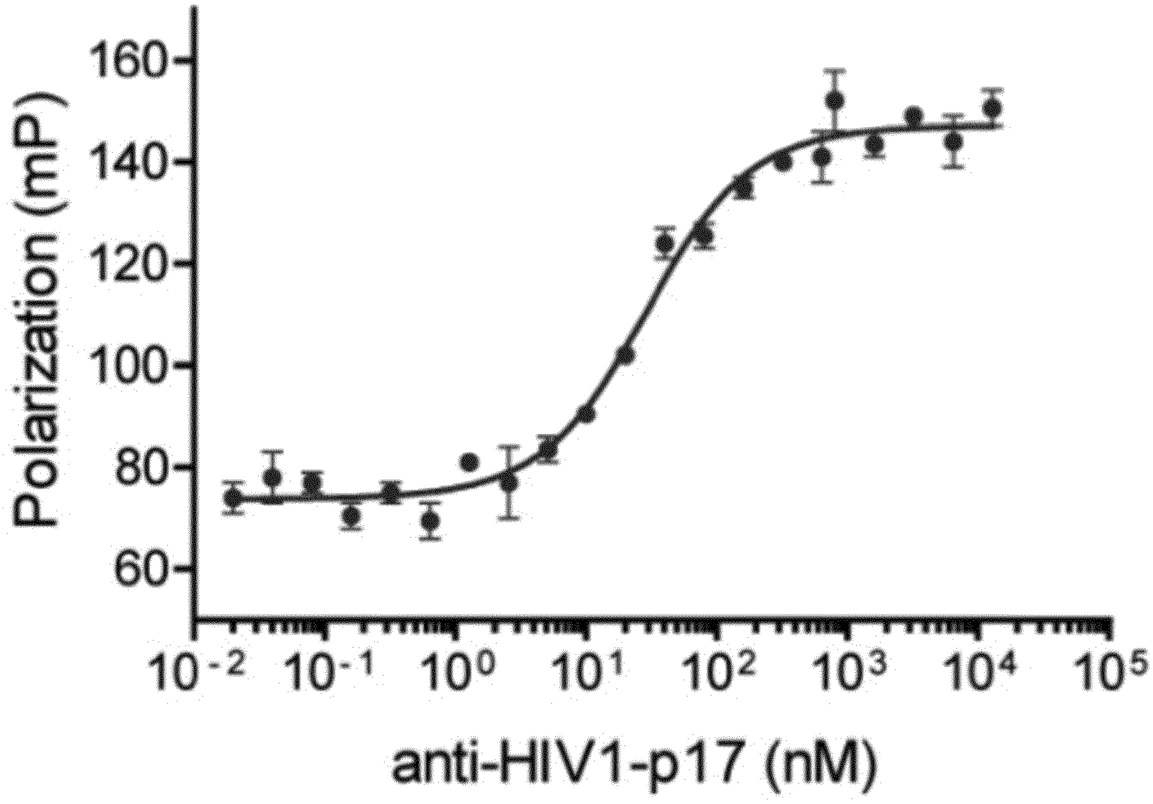
Polarization (mP)
160
140
120
100
80
60
$10^{-2}$ $10^{-1}$ $10^{0}$ $10^{1}$ $10^{2}$ $10^{3}$ $10^{4}$ $10^{5}$
anti-HIV1-p17 (nM)

**Fig. 11**

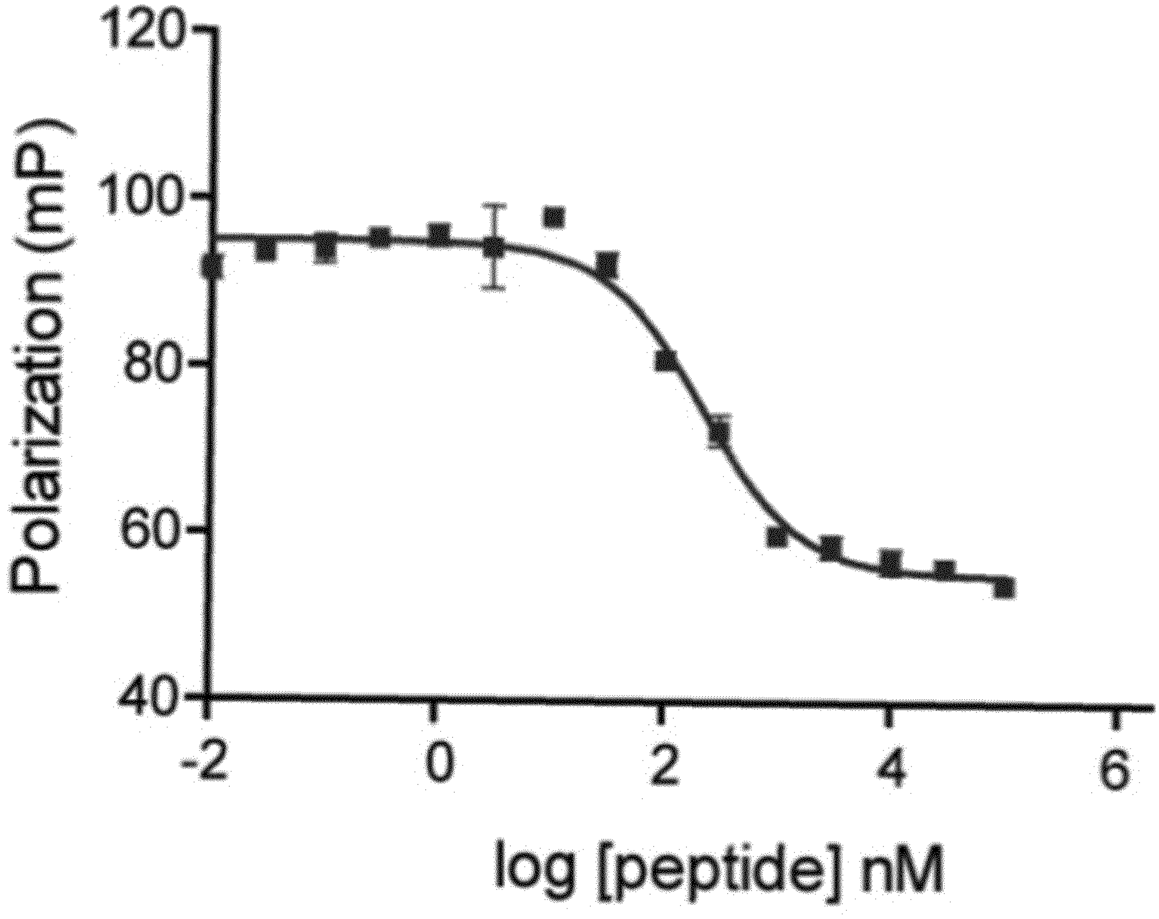


Fig. 12

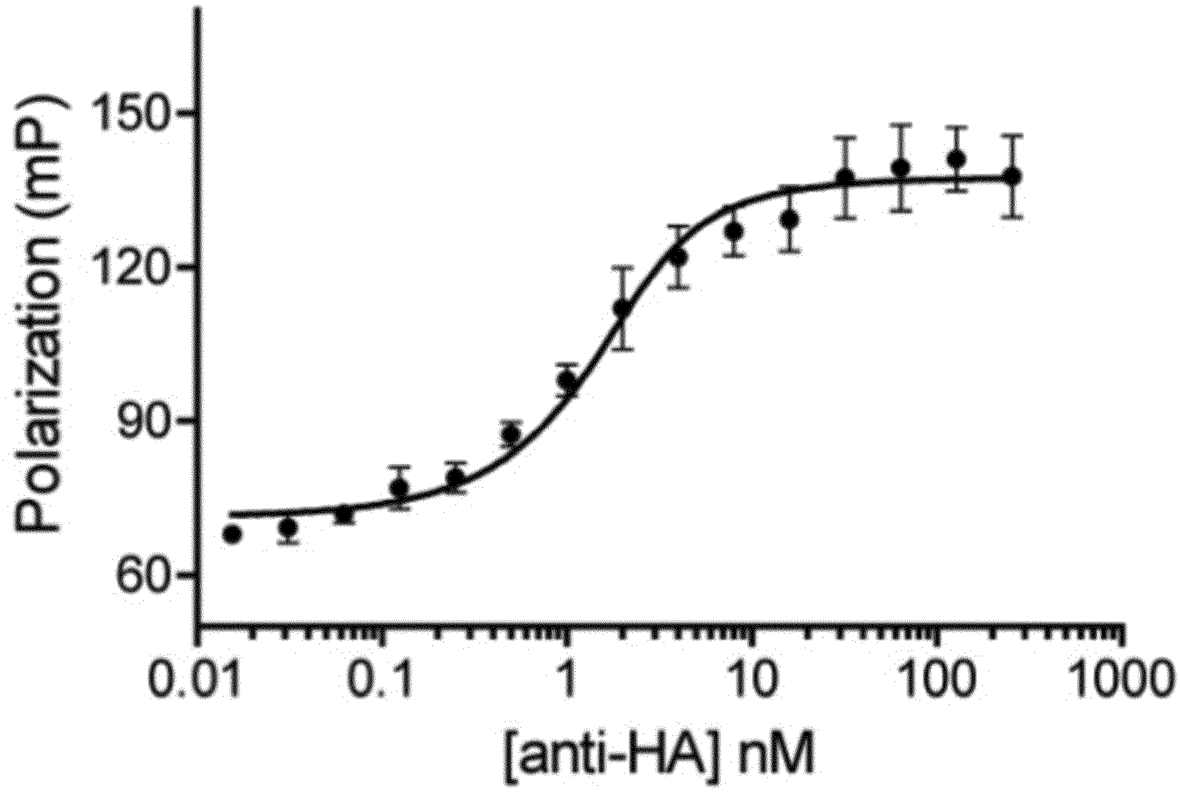


**Fig. 13**

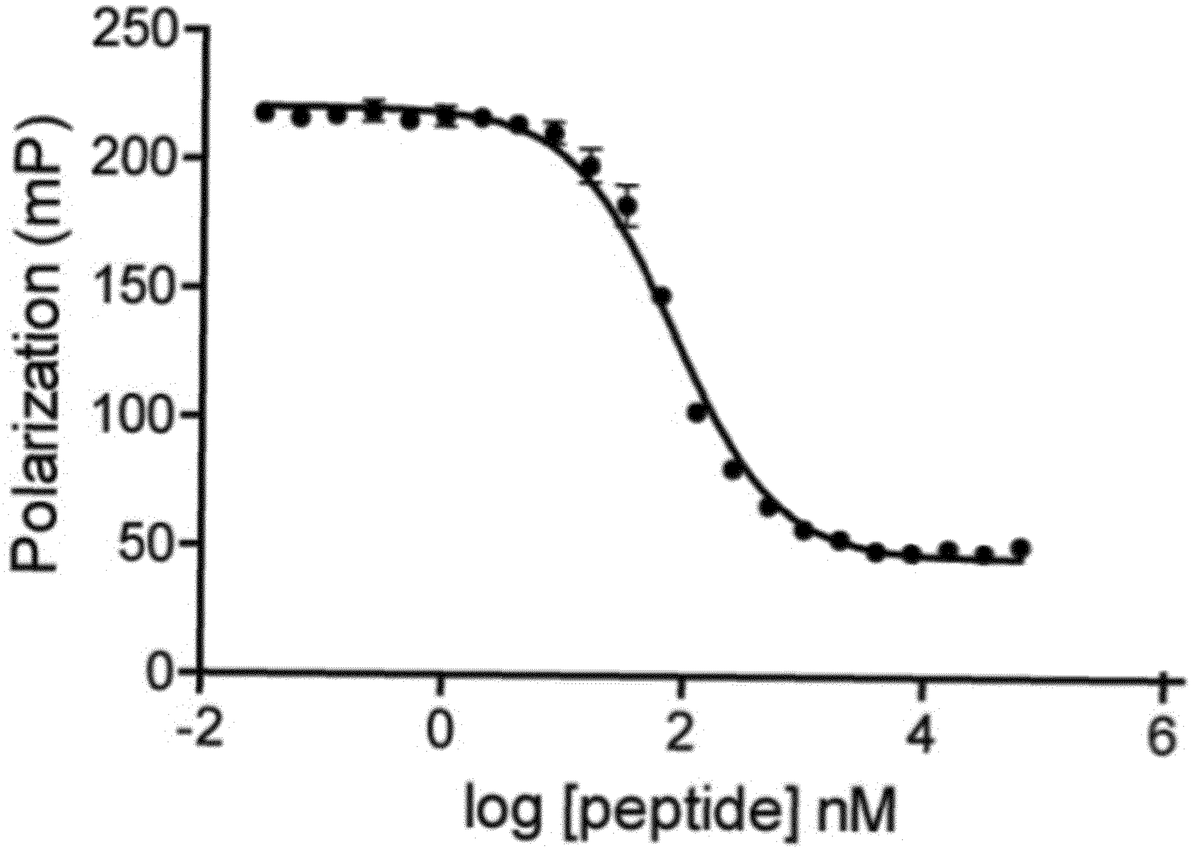


Fig. 14

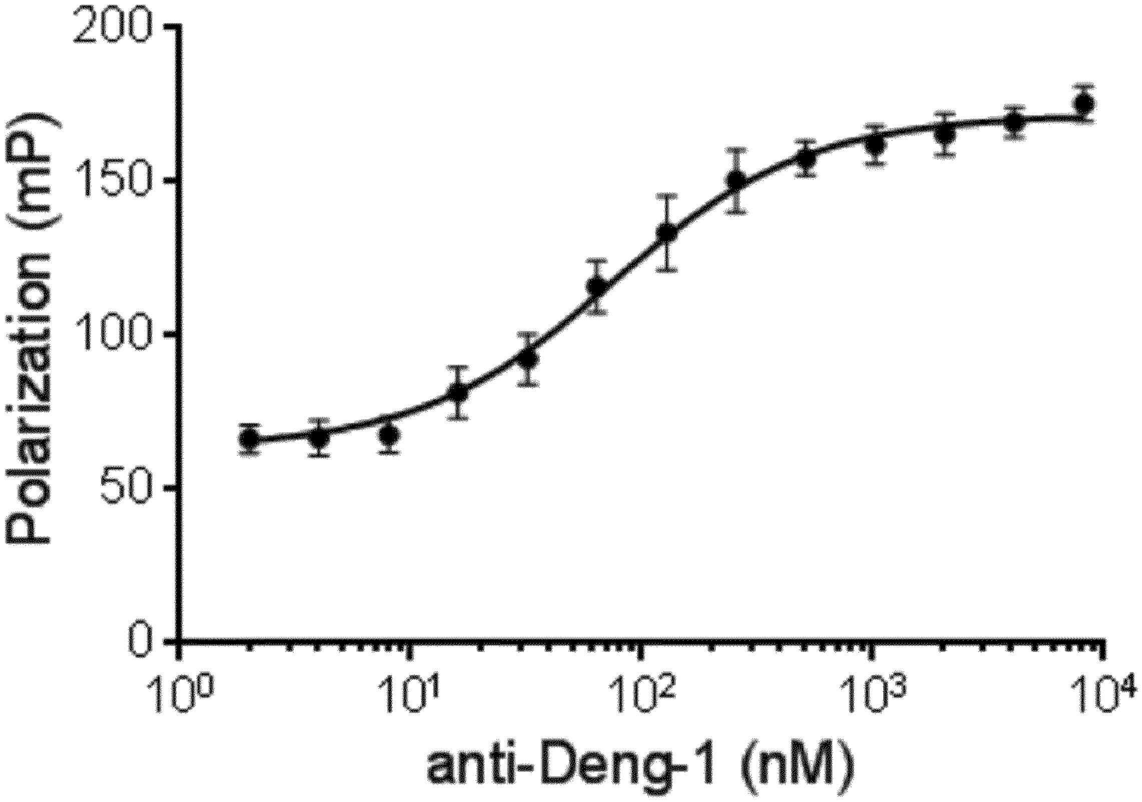
200
150
100
50
0
Polarization (mP)
10^0
10^1
10^2
10^3
10^4
anti-Deng-1 (nM)

Fig. 15

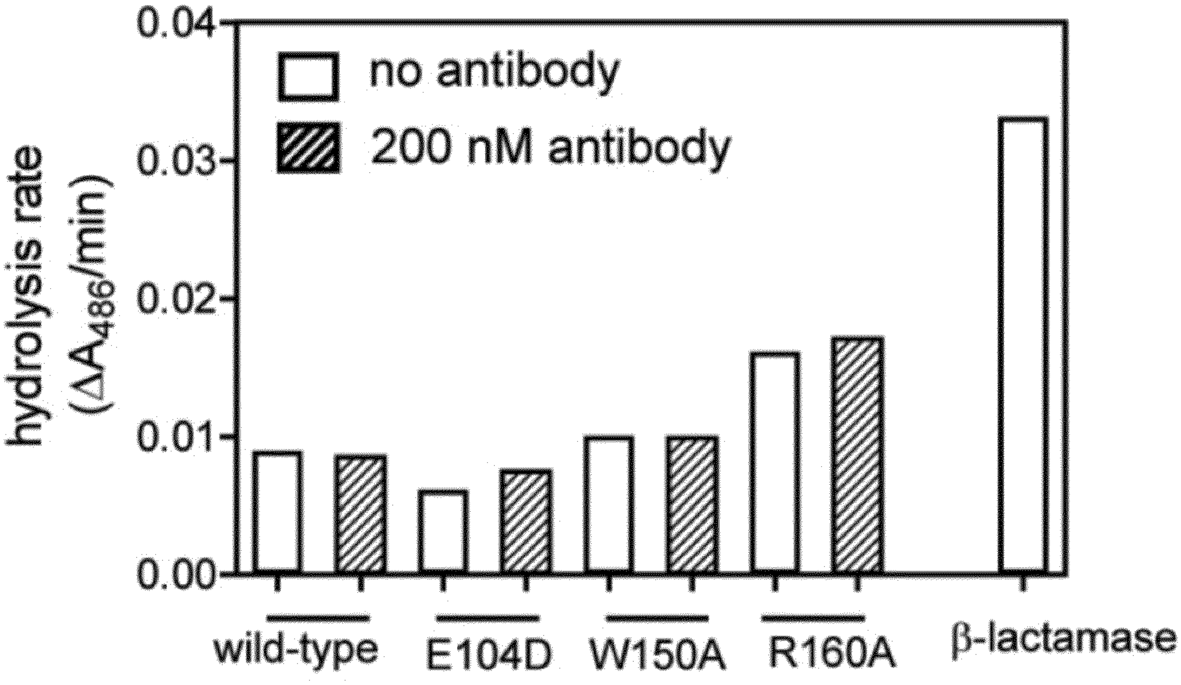
0.04
0.03
0.02
0.01
0.00
hydrolysis rate
(ΔA486/min)
no antibody
200 nM antibody
wild-type
E104D
W150A
R160A
β-lactamase

Fig. 16

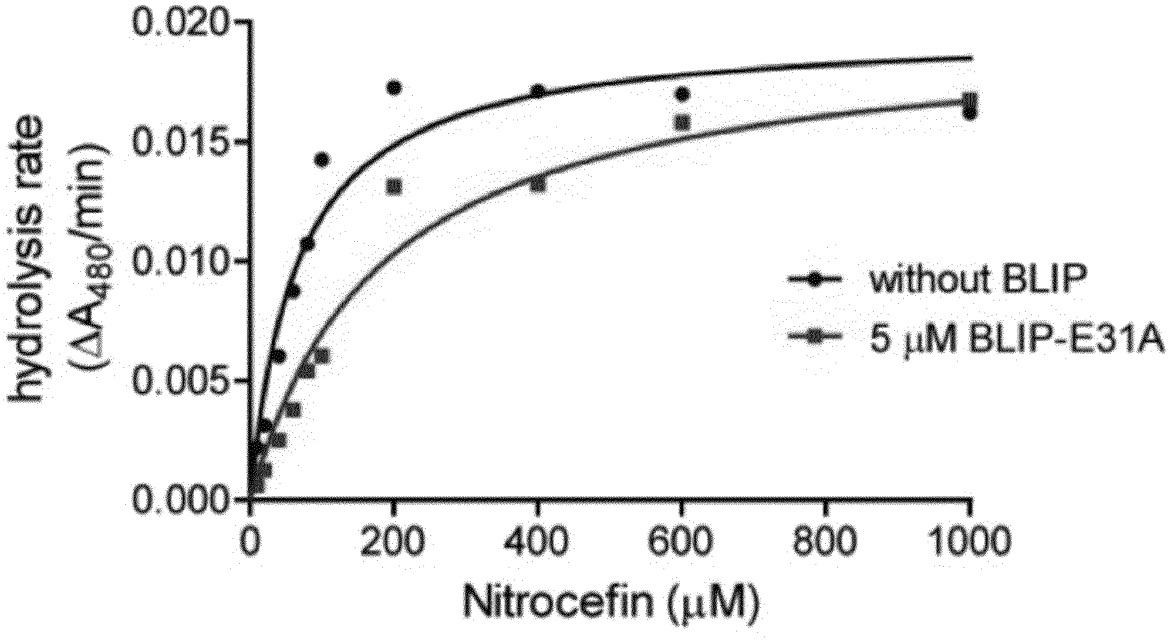
0.020
0.015
0.010
0.005
0.000
hydrolysis rate
(ΔA480/min)
0
200
400
600
800
1000
Nitrocefin (μM)
without BLIP
5 μM BLIP-E31A

Fig. 17

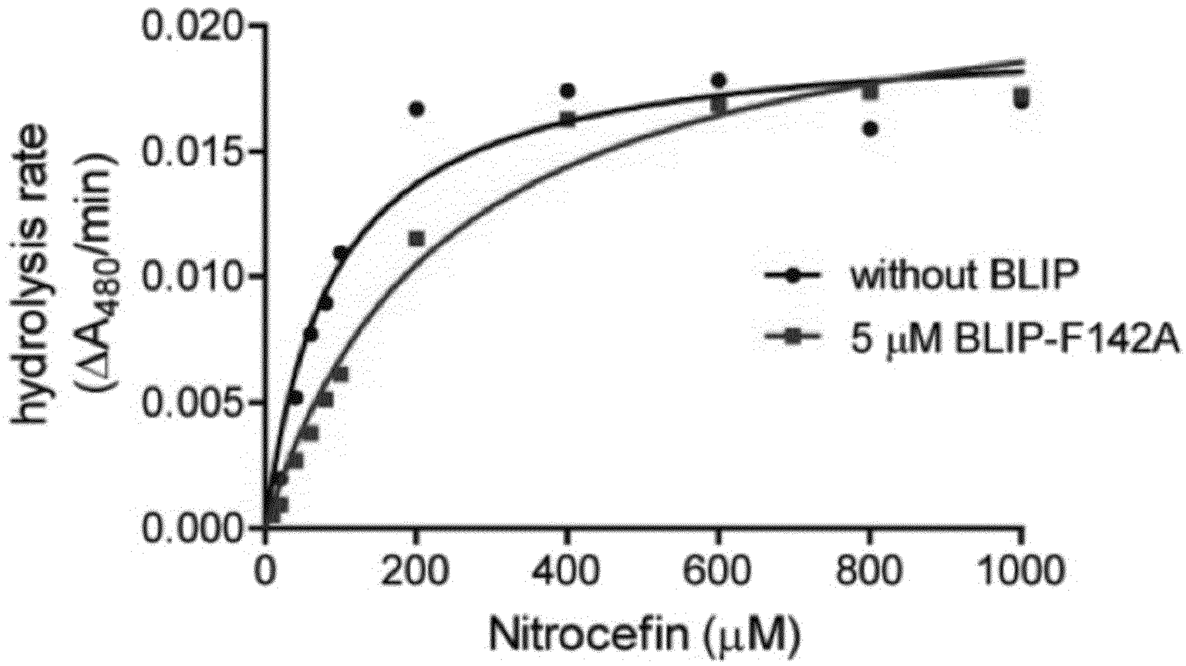
0.020
0.015
0.010
0.005
0.000
hydrolysis rate
(ΔA480/min)
0
200
400
600
800
1000
Nitrocefin (μM)
without BLIP
5 μM BLIP-F142A

**Fig. 18**

| Primer | Sequence |
|---|---|
| E104D-FW (for β-lactamase mutation) | CAGAATGATCTGGTGGAATATAGTCCGGTTACGG |
| E31A-FW | CGGAAAACTGCGCAACCGGCGGTAGC |
| F36A-FW | GCGAAACCGGCGGTAGCGCTGGCGATTC |
| D49A-FW | GCCACGCGGCCGGTGCTTATTACGCCTATG |
| Y53F-FW | GGTGATTATTACGCCTTTGCAACCTTTGGTTTCACC |
| E73M-FW | GCGGATGCAAAAGTGGATAGTAAAAGCCAGATGAAACTGCTG |
| K74A-FW | CCAGGAAGCCCTGCTGGCCCCGAGTGCACC |
| W112A-FW | GGGTAGTTGCACCACGGCCAGCGAATATTATCCGGC |
| F142A-FW | GCTATAGCTCTACCGGCGCTTACCGTGGTTCTGCG |
| F142V-FW | CTATAGCTCTACCGGCGTTTACCGTGGTTCTGC |
| F142Y-FW | CTATAGCTCTACCGGCTATTACCGTGGTTCTGC |
| H148A-FW | CGTGGTTCTGCGGCTCTGTGGTTCACGGATG |
| W150A-FW | CGTGGTTCTGCGCATCTGGCTTTCACGGATGGCG |
| W150F-FW | GGTTCTGCGCATCTGTTCTTCACGGATGGCGTG |
| R160A-FW | CGTGCTGCAAGGTAAAGCCCAGTGGGATCTGG |

Fig. 19

| Primer | Sequence |
|---|---|
| HA-open-FW | GCCGGATTACGCGGGCGGTTCCCATGGAG |
| HA-open-RW | GCACATCGTACGGATAACCACTAGTCCAATGTTTAATCAGG |
| HA-linker-FW | TATCCGTACGATGTGCCGGATTACGCGGGTGGAAGTGGGGGCAG |
| HA-linker-RW | CGCGTAATCCGGCACATCGTACGGATACTCACCTCCGGAACCG |
| Deng1-open-FW | GAACATAAATATAGCTGGAAAAGCGGTGGAAGTGGGGGCAG |
| Deng1-open-RW | CCGCTTTTCCAGCTATATTTATGTTCTAACTCACCTCCGGAACCG |
| Deng1-linker-FW | GAACATAAATATAGCTGGAAAAGCGGCGGTTCCCATGGAG |
| Deng1-linker-RW | GCTTTTCCAGCTATATTTATGTTCGCCACCACTAGTCCAATGTTTAATC |

# Fig. 20

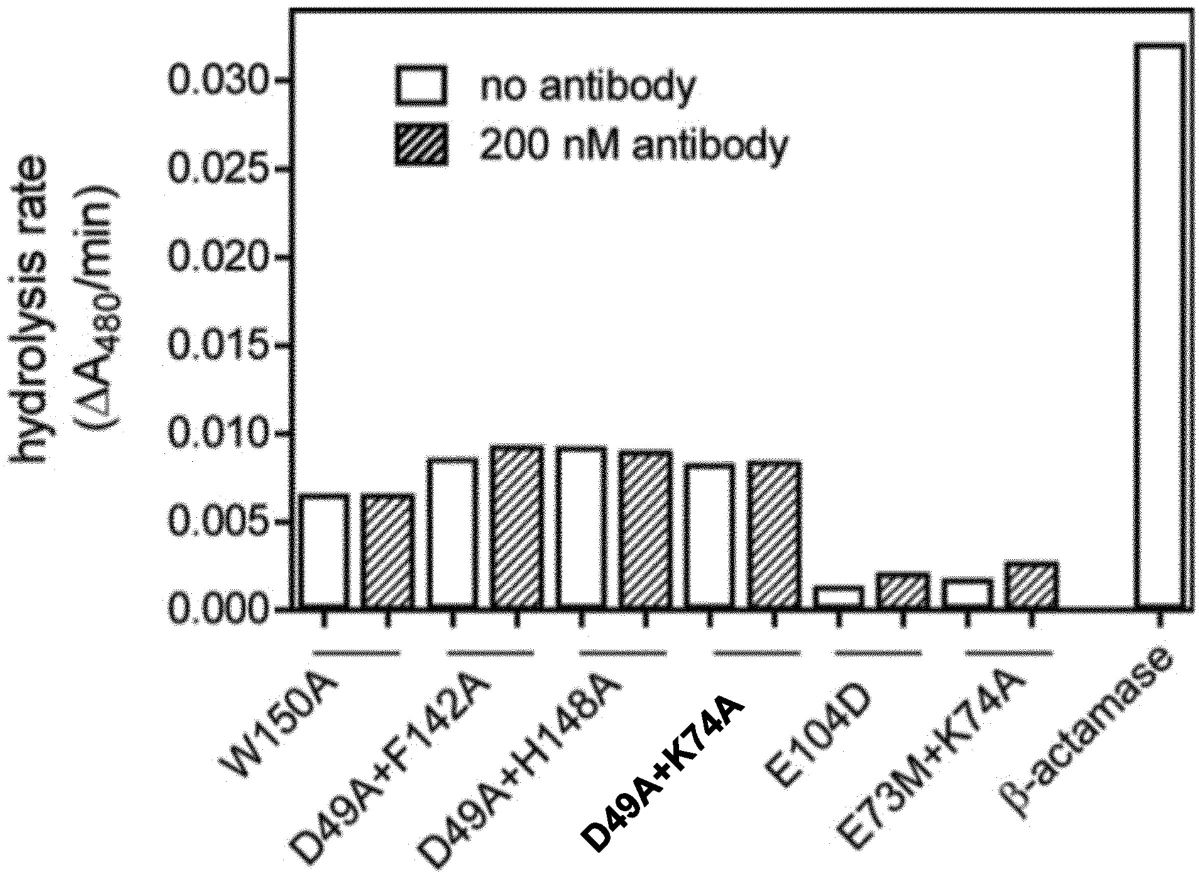
hydrolysis rate
($\Delta A_{480}$/min)
0.030
0.025
0.020
0.015
0.010
0.005
0.000
no antibody
200 nM antibody
W150A
D49A+F142A
D49A+H148A
D49A+K74A
E104D
E73M+K74A
β-actamase

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61706186 B

### Non-patent literature cited in the description


- **GOLYNSKIY, M. V. ; RURUP, W. F. ; MERKX, M.** Antibody detection by using a FRET-based protein conformational switch. *ChemBioChem,* 2010, vol. 11, 2264-2267
- **ZHANG, Z. ; PALZKILL, T.** Determinants of binding affinity and specificity for the interaction of TEM-1 and SME-1 beta-lactamase with beta-lactamase inhibitory protein. *The Journal of biological chemistry,* 2003, vol. 278, 45706-45712
- **NATALIE C.J. STRYNADKA ; SUSAN E. JENSEN ; PEDRO M. ALZARI ; MICHAEL N.G. JAMES.** A potent new mode of beta-lactamase inhibition revealed by the TEM-1/BLIP complex at 1.7 A resolution. *Nature Structural & Molecular Biology,* 1996, vol. 3, 290-297
- **HANES, M. S. ; REYNOLDS, K. A. ; MCNAMARA, C. ; GHOSH, P. ; BONOMO, R. A. ; KIRSCH, J. F. ; HANDEL, T. M.** Specificity and cooperativity at beta-lactamase position 104 in TEM-1/BLIP and SHV-1/BLIP interactions. *Proteins,* 2011, vol. 79, 1267-1276
- **QUAN, J. ; TIAN, J.** Circular polymerase extension cloning of complex gene libraries and pathways. *PLoS One,* 2009, vol. 4, e6441

SEQUENCE LISTING

<110> Technische Universiteit Eindhoven

<120> Switchable Reporter Enzymes for Homogenous Antibody Detection

<130> p109027pc00

<150> US 61/706186
<151> 2012-09-27

<160> 71

<170> PatentIn version 3.5

<210> 1
<211> 7
<212> PRT
<213> Unknown

<220>
<223> synthetic contruct

<400> 1

```
Trp Glu Lys Ile Arg Leu Arg
1               5
```

<210> 2
<211> 16
<212> PRT
<213> Unknown

<220>

<223> synthetic construct

<400> 2

```
Glu Leu Asp Arg Trp Glu Lys Ile Arg Leu Arg Pro Gly Gly Gly Cys
1               5                   10                  15
```

<210> 3
<211> 12
<212> PRT
<213> Unknown

<220>
<223> synthetic construct

<400> 3

```
Glu Leu Asp Arg Trp Glu Lys Ile Arg Leu Arg Pro
1               5                   10
```

<210> 4
<211> 13
<212> PRT
<213> Unknown

<220>
<223> synthetic construct

<400> 4

```
Tyr Pro Tyr Asp Val Pro Asp Tyr Ala Gly Gly Gly Cys
1               5                   10
```

<210> 5
<211> 9
<212> PRT
<213> Unknown

<220>
<223> synthetic contruct

<400> 5

```
Tyr Pro Tyr Asp Val Pro Asp Tyr Ala
1               5
```

<210> 6
<211> 12
<212> PRT
<213> Unknown

<220>
<223> synthetic construct

<400> 6

```
Glu His Lys Tyr Ser Trp Lys Ser Gly Gly Gly Cys
1               5                   10
```

<210> 7
<211> 5
<212> PRT
<213> Unknown

<220>
<223> synthetic construct

<400> 7

Glu Ala Ala Ala Lys
1               5

<210> 8
<211> 6
<212> PRT
<213> Unknown

<220>
<223> synthetic construct

<400> 8

Arg Arg Gly His Tyr Tyr
1               5

<210> 9
<211> 9
<212> PRT
<213> Unknown

<220>
<223> synthetic construct

<400> 9

Tyr Pro Tyr Asp Val Pro Asp Tyr Ala
1               5

<210> 10
<211> 11
<212> PRT
<213> Unknown

<220>
<223> synthetic construct

<400> 10

Trp Glu Lys Ile Arg Leu Arg Gly Gly Gly Cys
1               5                   10

<210> 11
<211> 11
<212> PRT
<213> Unknown

<220>

<223> synthetic construct

<400> 11

```
Glu Leu Asp Arg Trp Glu Lys Ile Arg Leu Arg
1               5                   10
```

<210> 12
<211> 474
<212> PRT
<213> Unknown

<220>
<223> synthetic construct

<400> 12

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45
```

```
Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Trp Glu
```

```
    290                 295                 300

Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
                405                 410                 415

Ala Ala Ala Lys Ala Gly Ser Gly Gly Ser Gly Gly Ser Gly Gly Ser
            420                 425                 430

Gly Gly Ser Gly Gly Ser Gly Gly Trp Glu Lys Ile Arg Leu Arg Gly
        435                 440                 445

Gly Ser Gly Gly Ser His Gly Arg Arg Gly His Tyr Tyr Gly Glu Phe
    450                 455                 460

Gly Gly Trp Ser His Pro Gln Phe Glu Lys
465                 470
```

<210> 13
<211> 633
<212> PRT
<213> Unknown

<220>
<223> synthetic construct

<400> 13

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30
```

```
Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
```

275 280 285

Glu Ile Gly Ala Ser Leu Ile Lys His Trp Thr Ser Gly Gly Trp Glu
290 295 300

Lys Ile Arg Leu Arg Gly Gly Ser Gly Gly Ser Gly Gly Ser Gly Gly
305 310 315 320

Ser Gly Gly Ser Gly Gly Ser Gly Ala Glu Ala Ala Ala Lys Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Ala Gly Ser Gly Gly Ser Gly Gly Ser
355 360 365

Gly Gly Pro Gln Gly Ile Leu Gly Gln Gly Ser Gly Gly Ser Gly Gly
370 375 380

Ser Gly Gly Ser Gly Ala Glu Ala Ala Ala Lys Glu Ala Ala Ala Lys
385 390 395 400

Glu Ala Ala Ala Lys Glu Ala Ala Ala Lys Glu Ala Ala Ala Lys Glu
405 410 415

Ala Ala Ala Lys Ala Gly Ser Gly Gly Ser Gly Gly Ser Gly Gly Ser
420 425 430

Gly Gly Ser Gly Gly Ser Gly Gly Trp Glu Lys Ile Arg Leu Arg Gly
435 440 445

Gly Ser Gly Gly Ser His Gly Ala Gly Val Met Thr Gly Ala Lys Phe
450 455 460

Thr Gln Ile Gln Phe Gly Met Thr Arg Gln Gln Val Leu Asp Ile Ala
465 470 475 480

Gly Ala Glu Asn Cys Glu Thr Gly Gly Ser Phe Gly Asp Ser Ile His
485 490 495

Cys Arg Gly His Ala Ala Gly Asp Tyr Tyr Ala Tyr Ala Thr Phe Gly
500 505 510

Phe Thr Ser Ala Ala Ala Asp Ala Lys Val Asp Ser Lys Ser Gln Glu
515 520 525

```
Lys Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
            580                 585                 590

Ser Ser Thr Gly Phe Tyr Arg Gly Ser Ala His Leu Trp Phe Thr Asp
        595                 600                 605

Gly Val Leu Gln Gly Lys Arg Gln Trp Asp Leu Val Gly Glu Phe Gly
    610                 615                 620

Gly Trp Ser His Pro Gln Phe Glu Lys
625                 630
```

<210> 14
<211> 633
<212> PRT
<213> Unknown

<220>
<223> synthetic construct

<400> 14

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
```

```
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Trp Glu
    290                 295                 300

Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335

Ala Ala Lys Glu Ala Ala Ala Lys Glu Ala Ala Ala Lys Glu Ala Ala
            340                 345                 350
```

```
Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
                405                 410                 415

Ala Ala Ala Lys Ala Gly Ser Gly Gly Ser Gly Gly Ser Gly Gly Ser
            420                 425                 430

Gly Gly Ser Gly Gly Ser Gly Gly Trp Glu Lys Ile Arg Leu Arg Gly
        435                 440                 445

Gly Ser Gly Gly Ser His Gly Ala Gly Val Met Thr Gly Ala Lys Phe
    450                 455                 460

Thr Gln Ile Gln Phe Gly Met Thr Arg Gln Gln Val Leu Asp Ile Ala
465                 470                 475                 480

Gly Ala Glu Asn Cys Glu Thr Gly Gly Ser Phe Gly Asp Ser Ile His
                485                 490                 495

Cys Arg Gly His Ala Ala Gly Asp Tyr Tyr Ala Tyr Ala Thr Phe Gly
            500                 505                 510

Phe Thr Ser Ala Ala Ala Asp Ala Lys Val Asp Ser Lys Ser Gln Glu
        515                 520                 525

Lys Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
            580                 585                 590

Ser Ser Thr Gly Phe Tyr Arg Gly Ser Ala His Leu Ala Phe Thr Asp
        595                 600                 605
```

```
Gly Val Leu Gln Gly Lys Arg Gln Trp Asp Leu Val Gly Glu Phe Gly
    610                 615                 620

Gly Trp Ser His Pro Gln Phe Glu Lys
625                 630
```

<210> 15
<211> 633
<212> PRT
<213> Unknown

<220>
<223> synthetic construct

<400> 15

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175
```

```
Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Trp Glu
    290                 295                 300

Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
                405                 410                 415

Ala Ala Ala Lys Ala Gly Ser Gly Gly Ser Gly Gly Ser Gly Gly Ser
            420                 425                 430
```

Gly Gly Ser Gly Gly Ser Gly Gly Trp Glu Lys Ile Arg Leu Arg Gly
435 440 445

Gly Ser Gly Gly Ser His Gly Ala Gly Val Met Thr Gly Ala Lys Phe
450 455 460

Thr Gln Ile Gln Phe Gly Met Thr Arg Gln Gln Val Leu Asp Ile Ala
465 470 475 480

Gly Ala Glu Asn Cys Glu Thr Gly Gly Ser Phe Gly Asp Ser Ile His
485 490 495

Cys Arg Gly His Ala Ala Gly Ala Tyr Tyr Ala Tyr Ala Thr Phe Gly
500 505 510

Phe Thr Ser Ala Ala Ala Asp Ala Lys Val Asp Ser Lys Ser Gln Glu
515 520 525

Lys Leu Leu Ala Pro Ser Ala Pro Thr Leu Thr Leu Ala Lys Phe Asn
530 535 540

Gln Val Thr Val Gly Met Thr Arg Ala Gln Val Leu Ala Thr Val Gly
545 550 555 560

Gln Gly Ser Cys Thr Thr Trp Ser Glu Tyr Tyr Pro Ala Tyr Pro Ser
565 570 575

Thr Ala Gly Val Thr Leu Ser Leu Ser Cys Phe Asp Val Asp Gly Tyr
580 585 590

Ser Ser Thr Gly Ala Tyr Arg Gly Ser Ala His Leu Trp Phe Thr Asp
595 600 605

Gly Val Leu Gln Gly Lys Arg Gln Trp Asp Leu Val Gly Glu Phe Gly
610 615 620

Gly Trp Ser His Pro Gln Phe Glu Lys
625 630

<210> 16
<211> 633
<212> PRT
<213> Unknown

<220>
<223> synthetic construct

<400> 16

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255
```

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Trp Glu
290 295 300

Lys Ile Arg Leu Arg Gly Gly Ser Gly Gly Ser Gly Gly Ser Gly Gly
305 310 315 320

Ser Gly Gly Ser Gly Gly Ser Gly Ala Glu Ala Ala Ala Lys Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Ala Gly Ser Gly Gly Ser Gly Gly Ser
355 360 365

Gly Gly Pro Gln Gly Ile Leu Gly Gln Gly Ser Gly Gly Ser Gly Gly
370 375 380

Ser Gly Gly Ser Gly Ala Glu Ala Ala Ala Lys Glu Ala Ala Ala Lys
385 390 395 400

Glu Ala Ala Ala Lys Glu Ala Ala Ala Lys Glu Ala Ala Ala Lys Glu
405 410 415

Ala Ala Ala Lys Ala Gly Ser Gly Gly Ser Gly Gly Ser Gly Gly Ser
420 425 430

Gly Gly Ser Gly Gly Ser Gly Gly Trp Glu Lys Ile Arg Leu Arg Gly
435 440 445

Gly Ser Gly Gly Ser His Gly Ala Gly Val Met Thr Gly Ala Lys Phe
450 455 460

Thr Gln Ile Gln Phe Gly Met Thr Arg Gln Gln Val Leu Asp Ile Ala
465 470 475 480

Gly Ala Glu Asn Cys Glu Thr Gly Gly Ser Phe Gly Asp Ser Ile His
485 490 495

Cys Arg Gly His Ala Ala Gly Ala Tyr Tyr Ala Tyr Ala Thr Phe Gly
500 505 510

```
Phe Thr Ser Ala Ala Ala Asp Ala Lys Val Asp Ser Lys Ser Gln Glu
        515                 520                 525

Lys Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
            580                 585                 590

Ser Ser Thr Gly Phe Tyr Arg Gly Ser Ala Ala Leu Trp Phe Thr Asp
        595                 600                 605

Gly Val Leu Gln Gly Lys Arg Gln Trp Asp Leu Val Gly Glu Phe Gly
    610                 615                 620

Gly Trp Ser His Pro Gln Phe Glu Lys
625                 630
```

<210> 17
<211> 633
<212> PRT
<213> Unknown

<220>
<223> synthetic construct

<400> 17

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80
```

```
Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Trp Glu
    290                 295                 300

Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335
```

```
Ala Ala Lys Glu Ala Ala Ala Lys Glu Ala Ala Ala Lys Glu Ala Ala
            340                 345                 350

Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
                405                 410                 415

Ala Ala Ala Lys Ala Gly Ser Gly Gly Ser Gly Gly Ser Gly Gly Ser
            420                 425                 430

Gly Gly Ser Gly Gly Ser Gly Gly Trp Glu Lys Ile Arg Leu Arg Gly
        435                 440                 445

Gly Ser Gly Gly Ser His Gly Ala Gly Val Met Thr Gly Ala Lys Phe
    450                 455                 460

Thr Gln Ile Gln Phe Gly Met Thr Arg Gln Gln Val Leu Asp Ile Ala
465                 470                 475                 480

Gly Ala Glu Asn Cys Glu Thr Gly Gly Ser Phe Gly Asp Ser Ile His
                485                 490                 495

Cys Arg Gly His Ala Ala Gly Ala Tyr Tyr Ala Tyr Ala Thr Phe Gly
            500                 505                 510

Phe Thr Ser Ala Ala Ala Asp Ala Lys Val Asp Ser Lys Ser Gln Glu
        515                 520                 525

Ala Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
```

580 585 590

Ser Ser Thr Gly Phe Tyr Arg Gly Ser Ala His Leu Trp Phe Thr Asp
595 600 605

Gly Val Leu Gln Gly Lys Arg Gln Trp Asp Leu Val Gly Glu Phe Gly
610 615 620

Gly Trp Ser His Pro Gln Phe Glu Lys
625 630

<210> 18
<211> 633
<212> PRT
<213> Unknown

<220>
<223> synthetic construct

<400> 18

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160
```

```
Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Trp Glu
    290                 295                 300

Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
```

```
                405                 410                 415

Ala Ala Ala Lys Ala Gly Ser Gly Gly Ser Gly Gly Ser Gly Gly Ser
            420                 425                 430

Gly Gly Ser Gly Gly Ser Gly Gly Trp Glu Lys Ile Arg Leu Arg Gly
        435                 440                 445

Gly Ser Gly Gly Ser His Gly Ala Gly Val Met Thr Gly Ala Lys Phe
    450                 455                 460

Thr Gln Ile Gln Phe Gly Met Thr Arg Gln Gln Val Leu Asp Ile Ala
465                 470                 475                 480

Gly Ala Glu Asn Cys Glu Thr Gly Gly Ser Phe Gly Asp Ser Ile His
                485                 490                 495

Cys Arg Gly His Ala Ala Gly Asp Tyr Tyr Ala Tyr Ala Thr Phe Gly
            500                 505                 510

Phe Thr Ser Ala Ala Ala Asp Ala Lys Val Asp Ser Lys Ser Gln Met
        515                 520                 525

Ala Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
            580                 585                 590

Ser Ser Thr Gly Phe Tyr Arg Gly Ser Ala His Leu Trp Phe Thr Asp
        595                 600                 605

Gly Val Leu Gln Gly Lys Arg Gln Trp Asp Leu Val Gly Glu Phe Gly
    610                 615                 620

Gly Trp Ser His Pro Gln Phe Glu Lys
625                 630
```

<210> 19
<211> 633
<212> PRT

<213> Unknown

<220>
<223> synthetic construct

<400> 19

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
```

225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Trp Glu
290 295 300

Lys Ile Arg Leu Arg Gly Gly Ser Gly Gly Ser Gly Gly Ser Gly Gly
305 310 315 320

Ser Gly Gly Ser Gly Gly Ser Gly Ala Glu Ala Ala Ala Lys Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Ala Gly Ser Gly Gly Ser Gly Gly Ser
355 360 365

Gly Gly Pro Gln Gly Ile Leu Gly Gln Gly Ser Gly Gly Ser Gly Gly
370 375 380

Ser Gly Gly Ser Gly Ala Glu Ala Ala Ala Lys Glu Ala Ala Ala Lys
385 390 395 400

Glu Ala Ala Ala Lys Glu Ala Ala Ala Lys Glu Ala Ala Ala Lys Glu
405 410 415

Ala Ala Ala Lys Ala Gly Ser Gly Gly Ser Gly Gly Ser Gly Gly Ser
420 425 430

Gly Gly Ser Gly Gly Ser Gly Gly Trp Glu Lys Ile Arg Leu Arg Gly
435 440 445

Gly Ser Gly Gly Ser His Gly Ala Gly Val Met Thr Gly Ala Lys Phe
450 455 460

Thr Gln Ile Gln Phe Gly Met Thr Arg Gln Gln Val Leu Asp Ile Ala
465 470 475 480

```
Gly Ala Glu Asn Cys Glu Thr Gly Gly Ser Phe Gly Asp Ser Ile His
                485                 490                 495

Cys Arg Gly His Ala Ala Gly Asp Tyr Tyr Ala Tyr Ala Thr Phe Gly
            500                 505                 510

Phe Thr Ser Ala Ala Ala Asp Ala Lys Val Asp Ser Lys Ser Gln Glu
        515                 520                 525

Lys Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560

Gln Gly Ser Cys Thr Thr Trp Ser Glu Tyr Tyr Pro Ala Tyr Pro Ser
                565                 570                 575

Thr Ala Gly Val Thr Leu Ser Leu Ser Cys Phe Asp Val Asp Gly Tyr
            580                 585                 590

Ser Ser Thr Gly Phe Tyr Arg Gly Ser Ala His Leu Trp Phe Thr Asp
        595                 600                 605

Gly Val Leu Gln Gly Lys Arg Gln Trp Asp Leu Val Gly Glu Phe Gly
    610                 615                 620

Gly Trp Ser His Pro Gln Phe Glu Lys
625                 630
```

<210> 20
<211> 633
<212> PRT
<213> Unknown

<220>
<223> synthetic construct

<400> 20

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
```

50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Trp Glu
290 295 300

```
Lys Ile Arg Leu Arg Gly Gly Ser Gly Gly Ser Gly Gly Ser Gly Gly
305                 310                 315                 320

Ser Gly Gly Ser Gly Gly Ser Gly Ala Glu Ala Ala Ala Lys Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Ala Gly Ser Gly Gly Ser Gly Gly Ser
        355                 360                 365

Gly Gly Pro Gln Gly Ile Leu Gly Gln Gly Ser Gly Gly Ser Gly Gly
    370                 375                 380

Ser Gly Gly Ser Gly Ala Glu Ala Ala Ala Lys Glu Ala Ala Ala Lys
385                 390                 395                 400

Glu Ala Ala Ala Lys Glu Ala Ala Ala Lys Glu Ala Ala Ala Lys Glu
                405                 410                 415

Ala Ala Ala Lys Ala Gly Ser Gly Gly Ser Gly Gly Ser Gly Gly Ser
            420                 425                 430

Gly Gly Ser Gly Gly Ser Gly Gly Trp Glu Lys Ile Arg Leu Arg Gly
        435                 440                 445

Gly Ser Gly Gly Ser His Gly Ala Gly Val Met Thr Gly Ala Lys Phe
    450                 455                 460

Thr Gln Ile Gln Phe Gly Met Thr Arg Gln Gln Val Leu Asp Ile Ala
465                 470                 475                 480

Gly Ala Glu Asn Cys Glu Thr Gly Gly Ser Phe Gly Asp Ser Ile His
                485                 490                 495

Cys Arg Gly His Ala Ala Gly Asp Tyr Tyr Ala Tyr Ala Thr Phe Gly
            500                 505                 510

Phe Thr Ser Ala Ala Ala Asp Ala Lys Val Asp Ser Lys Ser Gln Glu
        515                 520                 525

Lys Leu Leu Ala Pro Ser Ala Pro Thr Leu Thr Leu Ala Lys Phe Asn
    530                 535                 540

Gln Val Thr Val Gly Met Thr Arg Ala Gln Val Leu Ala Thr Val Gly
545                 550                 555                 560
```

Gln Gly Ser Cys Thr Thr Trp Ser Glu Tyr Tyr Pro Ala Tyr Pro Ser
565 570 575

Thr Ala Gly Val Thr Leu Ser Leu Ser Cys Phe Asp Val Asp Gly Tyr
580 585 590

Ser Ser Thr Gly Ala Tyr Arg Gly Ser Ala His Leu Trp Phe Thr Asp
595 600 605

Gly Val Leu Gln Gly Lys Arg Gln Trp Asp Leu Val Gly Glu Phe Gly
610 615 620

Gly Trp Ser His Pro Gln Phe Glu Lys
625 630

<210> 21
<211> 629
<212> PRT
<213> Unknown

<220>
<223> synthetic construct

<400> 21

Met Ser Ile Gln His Phe Arg Val Ala Leu Ile Pro Phe Phe Ala Ala
1 5 10 15

Phe Cys Leu Pro Val Phe Ala His His His His His His Leu Val Pro
20 25 30

Arg Gly Ser His Pro Glu Thr Leu Val Lys Val Lys Asp Ala Glu Asp
35 40 45

Gln Leu Gly Ala Arg Val Gly Tyr Ile Glu Leu Asp Leu Asn Ser Gly
50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Glu Tyr Ser Pro Val Thr Glu Lys His Leu Thr Asp Gly Met Thr
115 120 125

```
Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380
```

```
Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Glu Leu Asp Arg Trp Glu Lys Ile Arg Leu Arg Pro Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Glu Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 22
<211> 629
<212> PRT
<213> Unknown

<220>
<223> synthetic construct

<400> 22

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205
```

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

```
Phe Gly Met Thr Arg Gln Gln Val Leu Asp Ile Ala Gly Ala Glu Asn
465                 470                 475                 480

Cys Ala Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 23
<211> 629
<212> PRT
<213> Unknown

<220>
<223> synthetic construct

<400> 23

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30
```

```
Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285
```

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val

530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 24
<211> 629
<212> PRT
<213> Unknown

<220>
<223> synthetic construct

<400> 24

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110
```

```
Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
```

355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Ala Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 25
<211> 629
<212> PRT
<213> Unknown

<220>
<223> synthetic construct

<400> 25

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Glu Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
```

```
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430
```

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln
595 600 605

Gly Lys Ala Gln Trp Asp Leu Val Gly Glu Phe Gly Gly Trp Ser His
610 615 620

Pro Gln Phe Glu Lys
625

<210> 26
<211> 629
<212> PRT
<213> Unknown

<220>
<223> synthetic construct

<400> 26

Met Ser Ile Gln His Phe Arg Val Ala Leu Ile Pro Phe Phe Ala Ala

```
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255
```

```
Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Glu Leu Asp Arg Trp Glu Lys Ile Arg Leu Arg Pro Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Glu Thr Gly Gly Ser Ala Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510
```

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 27
<211> 629
<212> PRT
<213> Unknown

<220>
<223> synthetic construct

<400> 27

Met Ser Ile Gln His Phe Arg Val Ala Leu Ile Pro Phe Phe Ala Ala
1 5 10 15

Phe Cys Leu Pro Val Phe Ala His His His His His His Leu Val Pro
20 25 30

Arg Gly Ser His Pro Glu Thr Leu Val Lys Val Lys Asp Ala Glu Asp
35 40 45

Gln Leu Gly Ala Arg Val Gly Tyr Ile Glu Leu Asp Leu Asn Ser Gly
50 55 60

Lys Ile Leu Glu Ser Phe Arg Pro Glu Glu Arg Phe Pro Met Met Ser
65 70 75 80

```
Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335
```

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Phe Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 28
<211> 629
<212> PRT
<213> Unknown

<220>
<223> synthetic construct

<400> 28

Met Ser Ile Gln His Phe Arg Val Ala Leu Ile Pro Phe Phe Ala Ala
1 5 10 15

Phe Cys Leu Pro Val Phe Ala His His His His His His Leu Val Pro
20 25 30

Arg Gly Ser His Pro Glu Thr Leu Val Lys Val Lys Asp Ala Glu Asp
35 40 45

Gln Leu Gly Ala Arg Val Gly Tyr Ile Glu Leu Asp Leu Asn Ser Gly
50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

```
Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415
```

```
Ser Gly Gly Ser Gly Gly Ser Gly Gly Ser Gly Gly Ser Gly Gly Ser
                420                 425                 430

Gly Gly Glu Leu Asp Arg Trp Glu Lys Ile Arg Leu Arg Pro Gly Gly
            435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Glu Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Met Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 29
<211> 629
<212> PRT
<213> Unknown

<220>

<223> synthetic construct

<400> 29

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240
```

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His

```
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Ala Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 30
<211> 629
<212> PRT
<213> Unknown

<220>
<223> synthetic construct

<400> 30

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60
```

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser

305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

```
Thr Thr Ala Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 31
<211> 629
<212> PRT
<213> Unknown

<220>
<223> synthetic construct

<400> 31

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
```

```
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380
```

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Val Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 32
<211> 629
<212> PRT
<213> Unknown

<220>
<223> synthetic construct

<400> 32

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205
```

```
Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Glu Leu Asp Arg Trp Glu Lys Ile Arg Leu Arg Pro Gly Gly
        435                 440                 445

Ser His Gly Ala Gly Val Met Thr Gly Ala Lys Phe Thr Gln Ile Gln
    450                 455                 460
```

```
Phe Gly Met Thr Arg Gln Gln Val Leu Asp Ile Ala Gly Ala Glu Asn
465                 470                 475                 480

Cys Glu Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Tyr Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 33
<211> 629
<212> PRT
<213> Unknown

<220>
<223> synthetic construct

<400> 33

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30
```

```
Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285
```

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

```
Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Ala Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 34
<211> 629
<212> PRT
<213> Unknown

<220>
<223> synthetic construct

<400> 34

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110
```

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Glu Leu Asp Arg Trp Glu Lys Ile Arg Leu Arg Pro Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Glu Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Phe Phe Thr Asp Gly Val Leu Gln
        595                 600                 605

Gly Lys Arg Gln Trp Asp Leu Val Gly Glu Phe Gly Gly Trp Ser His

610 615 620

Pro Gln Phe Glu Lys
625

<210> 35
<211> 629
<212> PRT
<213> Unknown

<220>
<223> synthetic construct

<400> 35

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190
```

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu Leu
290 295 300

Asp Arg Trp Glu Lys Ile Arg Leu Arg Pro Gly Gly Ser Gly Gly Ser
305 310 315 320

Gly Gly Ser Gly Gly Ser Gly Gly Ser Gly Gly Ser Gly Ala Glu Ala
325 330 335

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly

```
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Glu Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Ala Gln Trp Asp Leu Val Gly Glu Phe Gly Gly Trp Ser His
    610                 615                 620

Pro Gln Phe Glu Lys
625
```

<210> 36
<211> 629
<212> PRT
<213> Unknown

<220>
<223> synthetic construct

<400> 36

```
Met Ser Ile Gln His Phe Arg Val Ala Leu Ile Pro Phe Phe Ala Ala
1               5                   10                  15
```

```
Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
```

```
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Glu Leu Asp Arg Trp Glu Lys Ile Arg Leu Arg Pro Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Ala Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510
```

```
Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 37
<211> 629
<212> PRT
<213> Unknown

<220>
<223> synthetic construct

<400> 37

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
```

```
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335
```

Ala Ala Lys Glu Ala Ala Ala Lys Glu Ala Ala Ala Lys Glu Ala Ala
340 345 350

Ala Lys Glu Ala Ala Ala Lys Glu Ala Ala Ala Lys Ala Gly Ser Gly
355 360 365

Gly Ser Gly Gly Ser Gly Gly Ser Gly Gly Ser Gly Gly Ser Gly Ala
370 375 380

Glu Ala Ala Ala Lys Glu Ala Ala Ala Lys Glu Ala Ala Ala Lys Glu
385 390 395 400

Ala Ala Ala Lys Glu Ala Ala Ala Lys Glu Ala Ala Ala Lys Ala Gly
405 410 415

Ser Gly Gly Ser Gly Gly Ser Gly Gly Ser Gly Gly Ser Gly Gly Ser
420 425 430

Gly Gly Glu Leu Asp Arg Trp Glu Lys Ile Arg Leu Arg Pro Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Glu Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Ala Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 38
<211> 617
<212> PRT
<213> Unknown

<220>
<223> synthetic construct

<400> 38

Met Ser Ile Gln His Phe Arg Val Ala Leu Ile Pro Phe Phe Ala Ala
1 5 10 15

Phe Cys Leu Pro Val Phe Ala His His His His His His Leu Val Pro
20 25 30

Arg Gly Ser His Pro Glu Thr Leu Val Lys Val Lys Asp Ala Glu Asp
35 40 45

Gln Leu Gly Ala Arg Val Gly Tyr Ile Glu Leu Asp Leu Asn Ser Gly
50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

```
Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Gly Gly
    290                 295                 300

Ser Gly Gly Ser Gly Gly Ser Gly Gly Ser Gly Gly Ser Gly Gly Ser
305                 310                 315                 320

Gly Ala Glu Ala Ala Ala Lys Glu Ala Ala Ala Lys Glu Ala Ala Ala
                325                 330                 335

Lys Glu Ala Ala Ala Lys Glu Ala Ala Ala Lys Glu Ala Ala Ala Lys
            340                 345                 350

Ala Gly Ser Gly Gly Ser Gly Gly Ser Gly Gly Ser Gly Gly Ser Gly
        355                 360                 365

Gly Ser Gly Ala Glu Ala Ala Ala Lys Glu Ala Ala Ala Lys Glu Ala
    370                 375                 380

Ala Ala Lys Glu Ala Ala Ala Lys Glu Ala Ala Ala Lys Glu Ala Ala
385                 390                 395                 400

Ala Lys Ala Gly Ser Gly Gly Ser Gly Gly Ser Gly Gly Ser Gly Gly
                405                 410                 415
```

Ser Gly Gly Ser Gly Gly Glu Leu Asp Arg Trp Glu Lys Ile Arg Leu
420 425 430

Arg Pro Gly Gly Ser His Gly Ala Gly Val Met Thr Gly Ala Lys Phe
435 440 445

Thr Gln Ile Gln Phe Gly Met Thr Arg Gln Gln Val Leu Asp Ile Ala
450 455 460

Gly Ala Glu Asn Cys Glu Thr Gly Gly Ser Phe Gly Asp Ser Ile His
465 470 475 480

Cys Arg Gly His Ala Ala Gly Asp Tyr Tyr Ala Tyr Ala Thr Phe Gly
485 490 495

Phe Thr Ser Ala Ala Ala Asp Ala Lys Val Asp Ser Lys Ser Gln Glu
500 505 510

Lys Leu Leu Ala Pro Ser Ala Pro Thr Leu Thr Leu Ala Lys Phe Asn
515 520 525

Gln Val Thr Val Gly Met Thr Arg Ala Gln Val Leu Ala Thr Val Gly
530 535 540

Gln Gly Ser Cys Thr Thr Trp Ser Glu Tyr Tyr Pro Ala Tyr Pro Ser
545 550 555 560

Thr Ala Gly Val Thr Leu Ser Leu Ser Cys Phe Asp Val Asp Gly Tyr
565 570 575

Ser Ser Thr Gly Ala Tyr Arg Gly Ser Ala His Leu Trp Phe Thr Asp
580 585 590

Gly Val Leu Gln Gly Lys Arg Gln Trp Asp Leu Val Gly Glu Phe Gly
595 600 605

Gly Trp Ser His Pro Gln Phe Glu Lys
610 615

<210> 39
<211> 617
<212> PRT
<213> Unknown

<220>
<223> synthetic construct

<400> 39

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255
```

```
Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Gly Glu Leu
    290                 295                 300

Asp Arg Trp Glu Lys Ile Arg Leu Arg Pro Gly Gly Ser Gly Gly Ser
305                 310                 315                 320

Gly Gly Ser Gly Gly Ser Gly Gly Ser Gly Gly Ser Gly Ala Glu Ala
                325                 330                 335

Ala Ala Lys Glu Ala Ala Ala Lys Glu Ala Ala Ala Lys Glu Ala Ala
            340                 345                 350

Ala Lys Glu Ala Ala Ala Lys Glu Ala Ala Ala Lys Ala Gly Ser Gly
        355                 360                 365

Gly Ser Gly Gly Ser Gly Gly Ser Gly Gly Ser Gly Gly Ser Gly Ala
    370                 375                 380

Glu Ala Ala Ala Lys Glu Ala Ala Ala Lys Glu Ala Ala Ala Lys Glu
385                 390                 395                 400

Ala Ala Ala Lys Glu Ala Ala Ala Lys Glu Ala Ala Ala Lys Ala Gly
                405                 410                 415

Ser Gly Gly Ser Gly Gly Ser Gly Gly Ser Gly Gly Ser Gly Gly Ser
            420                 425                 430

Gly Gly Gly Gly Ser His Gly Ala Gly Val Met Thr Gly Ala Lys Phe
        435                 440                 445

Thr Gln Ile Gln Phe Gly Met Thr Arg Gln Gln Val Leu Asp Ile Ala
    450                 455                 460

Gly Ala Glu Asn Cys Glu Thr Gly Gly Ser Phe Gly Asp Ser Ile His
465                 470                 475                 480

Cys Arg Gly His Ala Ala Gly Asp Tyr Tyr Ala Tyr Ala Thr Phe Gly
                485                 490                 495

Phe Thr Ser Ala Ala Ala Asp Ala Lys Val Asp Ser Lys Ser Gln Glu
            500                 505                 510
```

```
Lys Leu Leu Ala Pro Ser Ala Pro Thr Leu Thr Leu Ala Lys Phe Asn
        515                 520                 525

Gln Val Thr Val Gly Met Thr Arg Ala Gln Val Leu Ala Thr Val Gly
    530                 535                 540

Gln Gly Ser Cys Thr Thr Trp Ser Glu Tyr Tyr Pro Ala Tyr Pro Ser
545                 550                 555                 560

Thr Ala Gly Val Thr Leu Ser Leu Ser Cys Phe Asp Val Asp Gly Tyr
                565                 570                 575

Ser Ser Thr Gly Ala Tyr Arg Gly Ser Ala His Leu Trp Phe Thr Asp
            580                 585                 590

Gly Val Leu Gln Gly Lys Arg Gln Trp Asp Leu Val Gly Glu Phe Gly
        595                 600                 605

Gly Trp Ser His Pro Gln Phe Glu Lys
    610                 615
```

<210> 40
<211> 629
<212> PRT
<213> Unknown

<220>
<223> synthetic construct

<400> 40

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95
```

```
Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
                245                 250                 255

Ala Ala Leu Gly Pro Asp Gly Lys Pro Ser Arg Ile Val Val Ile Tyr
            260                 265                 270

Thr Thr Gly Ser Gln Ala Thr Met Asp Glu Arg Asn Arg Gln Ile Ala
        275                 280                 285

Glu Ile Gly Ala Ser Leu Ile Lys His Trp Thr Ser Gly Tyr Pro Tyr
    290                 295                 300

Asp Val Pro Asp Tyr Ala Gly Gly Ser Gly Gly Ser Gly Gly Gly Ser
305                 310                 315                 320

Gly Gly Ser Gly Ala Glu Ala Ala Ala Lys Glu Ala Ala Ala Lys Glu
                325                 330                 335

Ala Ala Ala Lys Glu Ala Ala Ala Lys Glu Ala Ala Ala Lys Glu Ala
            340                 345                 350
```

Ala Ala Lys Ala Gly Ser Gly Gly Ser Gly Gly Ser Gly Gly Pro Gln
355 360 365

Gly Ile Leu Gly Gln Gly Ser Gly Gly Ser Gly Gly Ser Gly Gly Ser
370 375 380

Gly Ala Glu Ala Ala Ala Lys Glu Ala Ala Ala Lys Glu Ala Ala Ala
385 390 395 400

Lys Glu Ala Ala Ala Lys Glu Ala Ala Ala Lys Glu Ala Ala Ala Lys
405 410 415

Ala Gly Ser Gly Gly Ser Gly Gly Ser Gly Gly Ser Gly Gly Ser Gly
420 425 430

Gly Ser Gly Gly Glu Tyr Pro Tyr Asp Val Pro Asp Tyr Ala Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Ala Thr Gly Gly Ser Phe Gly Asp Ser Ile His Cys Arg Gly His
485 490 495

Ala Ala Gly Asp Tyr Tyr Ala Tyr Ala Thr Phe Gly Phe Thr Ser Ala
500 505 510

Ala Ala Asp Ala Lys Val Asp Ser Lys Ser Gln Glu Lys Leu Leu Ala
515 520 525

Pro Ser Ala Pro Thr Leu Thr Leu Ala Lys Phe Asn Gln Val Thr Val
530 535 540

Gly Met Thr Arg Ala Gln Val Leu Ala Thr Val Gly Gln Gly Ser Cys
545 550 555 560

Thr Thr Trp Ser Glu Tyr Tyr Pro Ala Tyr Pro Ser Thr Ala Gly Val
565 570 575

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln

595 600 605

Gly Lys Arg Gln Trp Asp Leu Val Gly Glu Phe Gly Gly Trp Ser His
610 615 620

Pro Gln Phe Glu Lys
625

<210> 41
<211> 629
<212> PRT
<213> Unknown

<220>
<223> synthetic construct

<400> 41

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175
```

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Tyr Pro Tyr
290 295 300

Asp Val Pro Asp Tyr Ala Gly Gly Ser Gly Gly Ser Gly Gly Gly Ser
305 310 315 320

Gly Gly Ser Gly Ala Glu Ala Ala Ala Lys Glu Ala Ala Ala Lys Glu
325 330 335

Ala Ala Ala Lys Glu Ala Ala Ala Lys Glu Ala Ala Ala Lys Glu Ala
340 345 350

Ala Ala Lys Ala Gly Ser Gly Gly Ser Gly Gly Ser Gly Gly Pro Gln
355 360 365

Gly Ile Leu Gly Gln Gly Ser Gly Gly Ser Gly Gly Ser Gly Gly Ser
370 375 380

Gly Ala Glu Ala Ala Ala Lys Glu Ala Ala Ala Lys Glu Ala Ala Ala
385 390 395 400

Lys Glu Ala Ala Ala Lys Glu Ala Ala Ala Lys Glu Ala Ala Ala Lys
405 410 415

Ala Gly Ser Gly Gly Ser Gly Gly Ser Gly Gly Ser Gly Gly Ser Gly

```
                420                 425                 430

Gly Ser Gly Gly Glu Tyr Pro Tyr Asp Val Pro Asp Tyr Ala Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Ala Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 42
<211> 629
<212> PRT
<213> Unknown

<220>
<223> synthetic construct

<400> 42

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
65                  70                  75                  80

Thr Phe Lys Val Leu Leu Cys Gly Ala Val Leu Ser Arg Ile Asp Ala
                85                  90                  95

Gly Gln Glu Gln Leu Gly Arg Arg Ile His Tyr Ser Gln Asn Asp Leu
            100                 105                 110

Val Asp Tyr Ser Pro Val Thr Glu Lys His Leu Thr Asp Gly Met Thr
        115                 120                 125

Val Arg Glu Leu Cys Ser Ala Ala Ile Thr Met Ser Asp Asn Thr Ala
    130                 135                 140

Ala Asn Leu Leu Leu Thr Thr Ile Gly Gly Pro Lys Glu Leu Thr Ala
145                 150                 155                 160

Phe Leu His Asn Met Gly Asp His Val Thr Arg Leu Asp Arg Trp Glu
                165                 170                 175

Pro Glu Leu Asn Glu Ala Ile Pro Asn Asp Glu Arg Asp Thr Thr Met
            180                 185                 190

Pro Val Ala Met Ala Thr Thr Leu Arg Lys Leu Leu Thr Gly Glu Leu
        195                 200                 205

Leu Thr Leu Ala Ser Arg Gln Gln Leu Ile Asp Trp Met Glu Ala Asp
    210                 215                 220

Lys Val Ala Gly Pro Leu Leu Arg Ser Ala Leu Pro Ala Gly Trp Phe
225                 230                 235                 240

Ile Ala Asp Lys Ser Gly Ala Gly Glu Arg Gly Ser Arg Gly Ile Ile
```

245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu His
290 295 300

Lys Tyr Ser Trp Lys Ser Gly Gly Ser Gly Gly Ser Gly Gly Gly Ser
305 310 315 320

Gly Gly Ser Gly Ala Glu Ala Ala Ala Lys Glu Ala Ala Ala Lys Glu
325 330 335

Ala Ala Ala Lys Glu Ala Ala Ala Lys Glu Ala Ala Ala Lys Glu Ala
340 345 350

Ala Ala Lys Ala Gly Ser Gly Gly Ser Gly Gly Ser Gly Gly Pro Gln
355 360 365

Gly Ile Leu Gly Gln Gly Ser Gly Gly Ser Gly Gly Ser Gly Gly Ser
370 375 380

Gly Ala Glu Ala Ala Ala Lys Glu Ala Ala Ala Lys Glu Ala Ala Ala
385 390 395 400

Lys Glu Ala Ala Ala Lys Glu Ala Ala Ala Lys Glu Ala Ala Ala Lys
405 410 415

Ala Gly Ser Gly Gly Ser Gly Gly Ser Gly Gly Ser Gly Gly Ser Gly
420 425 430

Gly Ser Gly Gly Glu Leu Glu His Lys Tyr Ser Trp Lys Ser Gly Gly
435 440 445

Ser His Gly Ala Gly Val Met Thr Gly Ala Lys Phe Thr Gln Ile Gln
450 455 460

Phe Gly Met Thr Arg Gln Gln Val Leu Asp Ile Ala Gly Ala Glu Asn
465 470 475 480

Cys Ala Thr Gly Gly Ser Phe Gly Asp Ser Ile His Cys Arg Gly His
485 490 495

```
Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575

Thr Leu Ser Leu Ser Cys Phe Asp Val Asp Gly Tyr Ser Ser Thr Gly
            580                 585                 590

Phe Tyr Arg Gly Ser Ala His Leu Trp Phe Thr Asp Gly Val Leu Gln
        595                 600                 605

Gly Lys Arg Gln Trp Asp Leu Val Gly Glu Phe Gly Gly Trp Ser His
    610                 615                 620

Pro Gln Phe Glu Lys
625
```

<210> 43
<211> 629
<212> PRT
<213> Unknown

<220>
<223> synthetic construct

<400> 43

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
    50                  55                  60

Lys Ile Leu Glu Ser Phe Arg Pro Glu Glu Arg Phe Pro Met Met Ser
```

65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Thr Ser Gly Gly Glu His
290 295 300

Lys Tyr Ser Trp Lys Ser Gly Gly Ser Gly Gly Ser Gly Gly Gly Ser
305 310 315 320

```
Gly Gly Ser Gly Ala Glu Ala Ala Ala Lys Glu Ala Ala Ala Lys Glu
                325                 330                 335

Ala Ala Ala Lys Glu Ala Ala Ala Lys Glu Ala Ala Ala Lys Glu Ala
            340                 345                 350

Ala Ala Lys Ala Gly Ser Gly Gly Ser Gly Gly Ser Gly Gly Pro Gln
        355                 360                 365

Gly Ile Leu Gly Gln Gly Ser Gly Gly Ser Gly Gly Ser Gly Gly Ser
    370                 375                 380

Gly Ala Glu Ala Ala Ala Lys Glu Ala Ala Ala Lys Glu Ala Ala Ala
385                 390                 395                 400

Lys Glu Ala Ala Ala Lys Glu Ala Ala Ala Lys Glu Ala Ala Ala Lys
                405                 410                 415

Ala Gly Ser Gly Gly Ser Gly Gly Ser Gly Gly Ser Gly Gly Ser Gly
            420                 425                 430

Gly Ser Gly Gly Glu Leu Glu His Lys Tyr Ser Trp Lys Ser Gly Gly
        435                 440                 445

Ser His Gly Ala Gly Val Met Thr Gly Ala Lys Phe Thr Gln Ile Gln
    450                 455                 460

Phe Gly Met Thr Arg Gln Gln Val Leu Asp Ile Ala Gly Ala Glu Asn
465                 470                 475                 480

Cys Ala Thr Gly Gly Ser Phe Gly Asp Ser Ile His Cys Arg Gly His
                485                 490                 495

Ala Ala Gly Asp Tyr Tyr Ala Tyr Ala Thr Phe Gly Phe Thr Ser Ala
            500                 505                 510

Ala Ala Asp Ala Lys Val Asp Ser Lys Ser Gln Glu Lys Leu Leu Ala
        515                 520                 525

Pro Ser Ala Pro Thr Leu Thr Leu Ala Lys Phe Asn Gln Val Thr Val
    530                 535                 540

Gly Met Thr Arg Ala Gln Val Leu Ala Thr Val Gly Gln Gly Ser Cys
545                 550                 555                 560

Thr Thr Trp Ser Glu Tyr Tyr Pro Ala Tyr Pro Ser Thr Ala Gly Val
                565                 570                 575
```

Thr Leu Ser Leu Ser Cys Phe Asp Val Asp Gly Tyr Ser Ser Thr Gly
580 585 590

Phe Tyr Arg Gly Ser Ala His Leu Trp Phe Thr Asp Gly Val Leu Gln
595 600 605

Gly Lys Arg Gln Trp Asp Leu Val Gly Glu Phe Gly Gly Trp Ser His
610 615 620

Pro Gln Phe Glu Lys
625

<210> 44
<211> 298
<212> PRT
<213> Unknown

<220>
<223> synthetic construct

<400> 44

Met Ser Ile Gln His Phe Arg Val Ala Leu Ile Pro Phe Phe Ala Ala
1 5 10 15

Phe Cys Leu Pro Val Phe Ala His His His His His His Leu Val Pro
20 25 30

Arg Gly Ser His Pro Glu Thr Leu Val Lys Val Lys Asp Ala Glu Asp
35 40 45

Gln Leu Gly Ala Arg Val Gly Tyr Ile Glu Leu Asp Leu Asn Ser Gly
50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Glu Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp
290 295

<210> 45
<211> 310
<212> PRT
<213> Unknown

<220>
<223> synthetic construct

<400> 45

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser His Pro Glu Thr Leu Val Lys Val Lys Asp Ala Glu Asp
        35                  40                  45

Gln Leu Gly Ala Arg Val Gly Tyr Ile Glu Leu Asp Leu Asn Ser Gly
```

50 55 60

Lys Ile Leu Glu Ser Phe Arg Pro Glu Glu Arg Phe Pro Met Met Ser
65 70 75 80

Thr Phe Lys Val Leu Leu Cys Gly Ala Val Leu Ser Arg Ile Asp Ala
85 90 95

Gly Gln Glu Gln Leu Gly Arg Arg Ile His Tyr Ser Gln Asn Asp Leu
100 105 110

Val Asp Tyr Ser Pro Val Thr Glu Lys His Leu Thr Asp Gly Met Thr
115 120 125

Val Arg Glu Leu Cys Ser Ala Ala Ile Thr Met Ser Asp Asn Thr Ala
130 135 140

Ala Asn Leu Leu Leu Thr Thr Ile Gly Gly Pro Lys Glu Leu Thr Ala
145 150 155 160

Phe Leu His Asn Met Gly Asp His Val Thr Arg Leu Asp Arg Trp Glu
165 170 175

Pro Glu Leu Asn Glu Ala Ile Pro Asn Asp Glu Arg Asp Thr Thr Met
180 185 190

Pro Val Ala Met Ala Thr Thr Leu Arg Lys Leu Leu Thr Gly Glu Leu
195 200 205

Leu Thr Leu Ala Ser Arg Gln Gln Leu Ile Asp Trp Met Glu Ala Asp
210 215 220

Lys Val Ala Gly Pro Leu Leu Arg Ser Ala Leu Pro Ala Gly Trp Phe
225 230 235 240

Ile Ala Asp Lys Ser Gly Ala Gly Glu Arg Gly Ser Arg Gly Ile Ile
245 250 255

Ala Ala Leu Gly Pro Asp Gly Lys Pro Ser Arg Ile Val Val Ile Tyr
260 265 270

Thr Thr Gly Ser Gln Ala Thr Met Asp Glu Arg Asn Arg Gln Ile Ala
275 280 285

Glu Ile Gly Ala Ser Leu Ile Lys His Trp Glu Phe Gly Gly Trp Ser
290 295 300

His Pro Gln Phe Glu Lys
305 310

<210> 46
<211> 206
<212> PRT
<213> Unknown

<220>
<223> synthetic construct

<400> 46

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser Ser Gly Gly Ser His Gly Ala Gly Val Met Thr Gly Ala
        35                  40                  45

Lys Phe Thr Gln Ile Gln Phe Gly Met Thr Arg Gln Gln Val Leu Asp
    50                  55                  60

Ile Ala Gly Ala Glu Asn Cys Glu Thr Gly Gly Ser Phe Gly Asp Ser
65                  70                  75                  80

Ile His Cys Arg Gly His Ala Ala Gly Asp Tyr Tyr Ala Tyr Ala Thr
                85                  90                  95

Phe Gly Phe Thr Ser Ala Ala Ala Asp Ala Lys Val Asp Ser Lys Ser
            100                 105                 110

Gln Glu Lys Leu Leu Ala Pro Ser Ala Pro Thr Leu Thr Leu Ala Lys
        115                 120                 125

Phe Asn Gln Val Thr Val Gly Met Thr Arg Ala Gln Val Leu Ala Thr
    130                 135                 140

Val Gly Gln Gly Ser Cys Thr Thr Trp Ser Glu Tyr Tyr Pro Ala Tyr
145                 150                 155                 160

Pro Ser Thr Ala Gly Val Thr Leu Ser Leu Ser Cys Phe Asp Val Asp
                165                 170                 175

Gly Tyr Ser Ser Thr Gly Phe Tyr Arg Gly Ser Ala His Leu Trp Phe
            180                 185                 190

Thr Asp Gly Val Leu Gln Gly Lys Arg Gln Trp Asp Leu Val

        195                 200                 205
```

<210> 47
<211> 206
<212> PRT
<213> Unknown

<220>
<223> synthetic construct

<400> 47

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser Ser Gly Gly Ser His Gly Ala Gly Val Met Thr Gly Ala
        35                  40                  45

Lys Phe Thr Gln Ile Gln Phe Gly Met Thr Arg Gln Gln Val Leu Asp
    50                  55                  60

Ile Ala Gly Ala Glu Asn Cys Ala Thr Gly Gly Ser Phe Gly Asp Ser
65                  70                  75                  80

Ile His Cys Arg Gly His Ala Ala Gly Asp Tyr Tyr Ala Tyr Ala Thr
                85                  90                  95

Phe Gly Phe Thr Ser Ala Ala Ala Asp Ala Lys Val Asp Ser Lys Ser
            100                 105                 110

Gln Glu Lys Leu Leu Ala Pro Ser Ala Pro Thr Leu Thr Leu Ala Lys
        115                 120                 125

Phe Asn Gln Val Thr Val Gly Met Thr Arg Ala Gln Val Leu Ala Thr
    130                 135                 140

Val Gly Gln Gly Ser Cys Thr Thr Trp Ser Glu Tyr Tyr Pro Ala Tyr
145                 150                 155                 160

Pro Ser Thr Ala Gly Val Thr Leu Ser Leu Ser Cys Phe Asp Val Asp
                165                 170                 175

Gly Tyr Ser Ser Thr Gly Phe Tyr Arg Gly Ser Ala His Leu Trp Phe
            180                 185                 190

Thr Asp Gly Val Leu Gln Gly Lys Arg Gln Trp Asp Leu Val
        195                 200                 205
```

<210> 48
<211> 206
<212> PRT
<213> Unknown

<220>
<223> synthetic construct

<400> 48

```
Met Ser Ile Gln His Phe Arg Val Ala Leu Ile Pro Phe Phe Ala Ala
1               5                   10                  15

Phe Cys Leu Pro Val Phe Ala His His His His His His Leu Val Pro
            20                  25                  30

Arg Gly Ser Ser Gly Gly Ser His Gly Ala Gly Val Met Thr Gly Ala
        35                  40                  45

Lys Phe Thr Gln Ile Gln Phe Gly Met Thr Arg Gln Gln Val Leu Asp
    50                  55                  60

Ile Ala Gly Ala Glu Asn Cys Glu Thr Gly Gly Ser Phe Gly Asp Ser
65                  70                  75                  80

Ile His Cys Arg Gly His Ala Ala Gly Asp Tyr Tyr Ala Tyr Ala Thr
                85                  90                  95

Phe Gly Phe Thr Ser Ala Ala Ala Asp Ala Lys Val Asp Ser Lys Ser
            100                 105                 110

Gln Glu Lys Leu Leu Ala Pro Ser Ala Pro Thr Leu Thr Leu Ala Lys
        115                 120                 125

Phe Asn Gln Val Thr Val Gly Met Thr Arg Ala Gln Val Leu Ala Thr
    130                 135                 140

Val Gly Gln Gly Ser Cys Thr Thr Trp Ser Glu Tyr Tyr Pro Ala Tyr
145                 150                 155                 160

Pro Ser Thr Ala Gly Val Thr Leu Ser Leu Ser Cys Phe Asp Val Asp
                165                 170                 175

Gly Tyr Ser Ser Thr Gly Ala Tyr Arg Gly Ser Ala His Leu Trp Phe
            180                 185                 190

Thr Asp Gly Val Leu Gln Gly Lys Arg Gln Trp Asp Leu Val
        195                 200                 205
```

<210> 49
<211> 34
<212> DNA
<213> Unknown

<220>
<223> synthetic construct

<400> 49
cagaatgatc tggtggaata tagtccggtt acgg 34

<210> 50
<211> 26
<212> DNA
<213> Unknown

<220>
<223> synthetic contruct

<400> 50
cggaaaactg cgcaaccggc ggtagc 26

<210> 51
<211> 28
<212> DNA
<213> Unknown

<220>
<223> synthetic construct

<400> 51
gcgaaaccgg cggtagcgct ggcgattc 28

<210> 52
<211> 30
<212> DNA
<213> Unknown

<220>
<223> synthetic construct

<400> 52
gccacgcggc cggtgcttat tacgcctatg 30

<210> 53
<211> 36
<212> DNA
<213> Unknown

<220>
<223> synthetic construct

<400> 53
ggtgattatt acgcctttgc aacctttggt ttcacc 36

<210> 54
<211> 42
<212> DNA
<213> Unknown

<220>
<223> synthetic construct

<400> 54
gcggatgcaa aagtggatag taaaagccag atgaaactgc tg 42

<210> 55
<211> 30
<212> DNA
<213> Unknown

<220>
<223> synthetic construct

<400> 55
ccaggaagcc ctgctggccc cgagtgcacc 30

<210> 56
<211> 36
<212> DNA
<213> Unknown

<220>
<223> synthetic construct

<400> 56
gggtagttgc accacggcca gcgaatatta tccggc 36

<210> 57
<211> 35
<212> DNA
<213> Unknown

<220>
<223> synthetic construct

<400> 57
gctatagctc taccggcgct taccgtggtt ctgcg 35

<210> 58
<211> 33
<212> DNA
<213> Unknown

<220>
<223> synthetic construct

<400> 58
ctatagctct accggcgttt accgtggttc tgc 33

<210> 59
<211> 33
<212> DNA
<213> Unknown

<220>
<223> synthetic construct

<400> 59
ctatagctct accggctatt accgtggttc tgc 33

<210> 60
<211> 31
<212> DNA

<213> Unknown

<220>
<223> synthetic construct

<400> 60
cgtggttctg cggctctgtg gttcacggat g 31

<210> 61
<211> 34
<212> DNA
<213> Unknown

<220>
<223> synthetic construct

<400> 61
cgtggttctg cgcatctggc tttcacggat ggcg 34

<210> 62
<211> 33
<212> DNA
<213> Unknown

<220>
<223> synthetic construct

<400> 62
ggttctgcgc atctgttctt cacggatggc gtg 33

<210> 63
<211> 32
<212> DNA
<213> Unknown

<220>
<223> synthetic construct

<400> 63
cgtgctgcaa ggtaaagccc agtgggatct gg 32

<210> 64
<211> 29
<212> DNA
<213> Unknown

<220>
<223> synthetic construct

<400> 64
gccggattac gcgggcggtt cccatggag 29

<210> 65
<211> 41
<212> DNA
<213> Unknown

<220>

<223> synthetic construct

<400> 65
gcacatcgta cggataacca ctagtccaat gtttaatcag g 41

<210> 66
<211> 44
<212> DNA
<213> Unknown

<220>
<223> synthetic construct

<400> 66
tatccgtacg atgtgccgga ttacgcgggt ggaagtgggg gcag 44

<210> 67
<211> 43
<212> DNA
<213> Unknown

<220>
<223> synthetic construct

<400> 67
cgcgtaatcc ggcacatcgt acggatactc acctccggaa ccg 43

<210> 68
<211> 41
<212> DNA
<213> Unknown

<220>
<223> synthetic construct

<400> 68
gaacataaat atagctggaa aagcggtgga agtgggggca g 41

<210> 69
<211> 45
<212> DNA
<213> Unknown

<220>
<223> synthetic construct

<400> 69
ccgcttttcc agctatattt atgttctaac tcacctccgg aaccg 45

<210> 70
<211> 40
<212> DNA
<213> Unknown

<220>
<223> synthetic construct

<400> 70

gaacataaat atagctggaa aagcggcggt tcccatggag 40

<210> 71
<211> 49
<212> DNA
<213> Unknown

<220>
<223> synthetic construct

<400> 71
gcttttccag ctatatttat gttcgccacc actagtccaa tgtttaatc 49